# FASCICULE DE BREVET EUROPEEN

(11) **EP 2 756 305 B1**
(45) Date de publication et mention de la délivrance du brevet: **21.11.2018**
(21) Numéro de dépôt: 12767069.3
(22) Date de dépôt: 13.09.2012
(51) Int. Cl.: G01N 33/542

(54) **PROCEDE DE DETERMINATION DE LA GLYCOSYLATION D'UN ANTICORPS**
VERFAHREN ZUR BESTIMMUNG DER GLYCOSYLIERUNG EINES ANTIKÖRPERS
METHOD FOR DETERMINING THE GLYCOSYLATION OF AN ANTIBODY

(30) Priorité: 16.09.2011 FR 1158245
(43) Date de publication de la demande: 23.07.2014
(73) Titulaire: Cisbio Bioassays, 30200 Codolet (FR)
(72) Inventeur: JAGA, Delphine, 30150 Sauveterre (FR); MOKRANE, Hamed, F-30133 Les Angles (FR); MARTINEZ, Stéphane, F-30400 Villeneuve Les Avignon (FR); FINK, Michel, F-30200 Bagnols Sur Ceze (FR)
(74) Mandataire: Marro, Nicolas
(86) Numéro de dépôt international: PCT/FR2012/052049
(87) Numéro de publication internationale: WO 2013/038113

(56) Documents cités:
- EP-A1- 2 169 404
- WO-A1-2004/031404
- WO-A1-2007/080274
- WO-A2-2007/026099
- WO-A2-2011/018586

## Description

### Domaine de l'invention :

L'invention est relative à un procédé de détection de la liaison d'un anticorps à un récepteur des fragments cristallisables Fc (ci-après « récepteurs Fc » ou FcR) présents à la surface d'une cellule et également un procédé de détermination du niveau de glycosylation d'un anticorps.

### Etat de la technique

Les anticorps monoclonaux recombinants ont permis, depuis quelques années, une révolution thérapeutique. Bien que leur efficacité clinique ne soit plus à prouver, leur mode d'action chez les patients reste peu connu. L'effet thérapeutique des anticorps ciblant des antigènes membranaires passe notamment par le recrutement de cellules effectrices exprimant des récepteurs pour le fragment cristallisable des anticorps (ci-après, « récepteurs Fc »)

Les récepteurs Fc sont des protéines présentes à la surface de certaines cellules contribuant aux fonctions du système immunitaire, en particulier des cellules NK (« natural killer », tueuses naturelles), des macrophages, des neutrophiles et des mastocytes. Leur nom provient de leur capacité à se lier à la région Fc (fragment crystallisable) des anticorps. Il en existe plusieurs types, qui sont classés en fonction du type d'anticorps qu'ils reconnaissent : les récepteur Fc gamma (FcγR) se lient aux IgG, le récepteur Fc alpha (FcαR) se lie aux IgA et les récepteur Fc epsilon (FcεR) se lient aux IgE.

La liaison du récepteur Fc avec un anticorps déclenche différents mécanismes en fonction de la nature de la cellule sur laquelle est exprimé ce récepteur. La table 1 est un résumé de la distribution cellulaire des différents récepteurs Fc et des mécanismes déclenchés par la liaison du récepteur à un anticorps.

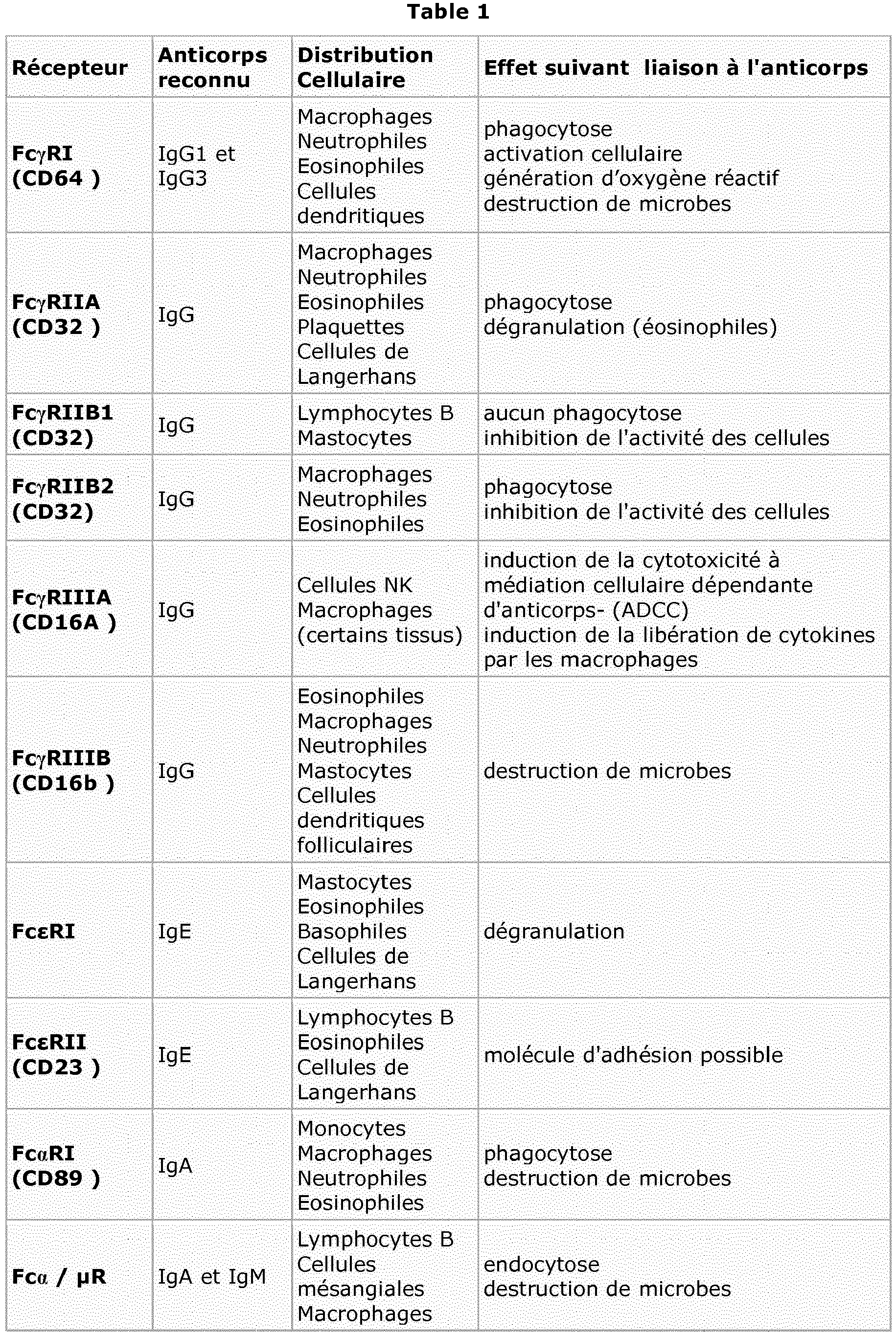

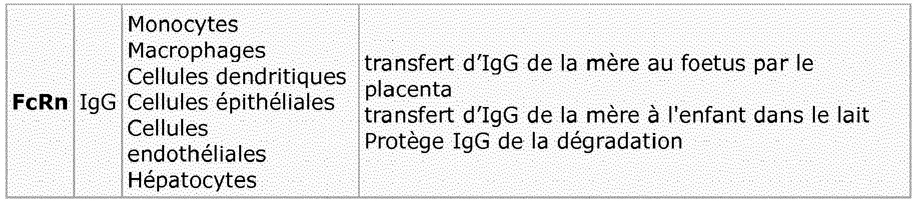

Au regard de l'importance des mécanismes liés à la liaison des récepteurs Fc aux anticorps, il serait particulièrement avantageux de pouvoir détecter cette liaison lorsque ces récepteurs sont dans un contexte cellulaire, en particulier dans le cadre du développement d'anticorps ou de fragments Fc à vocation diagnostique ou thérapeutique.

Des anticorps thérapeutiques déclenchant le mécanisme ADCC sont actuellement commercialisés (Herceptin®, Cetuximab®) et indiqués dans le traitement de certains cancers : le fragment Fc de ces anticorps se lie à un récepteur Fc présent sur les cellules NK, alors que les domaines variables de ces anticorps reconnaissent un antigène présent sur des cellules tumorales (Herceptin : Her2, Cetuximab : Her1). La liaison de ces anticorps à la fois à une cellule NK et à une cellule cancéreuse entraîne notamment la destruction de cette dernière par l'activation du mécanisme ADCC.

Il est par ailleurs connu que la glycosylation du fragment Fc des anticorps influence l'affinité de ces anticorps pour les FcR, et donc leur capacité à recruter des cellules effectrices. En particulier des anticorps thérapeutiques modifiés ne comportant pas ou peu de résidus fucoses au niveau des groupes N-Glycans du fragment Fc provoquent une forte réponse ADCC à de faibles concentrations et avec une bien meilleure efficacité en comparaison avec leurs équivalents fucosylés (Shields et al, J Biol Chem. 2002 Jul 26;277(30):26733-40, Suzuki et al Clin Cancer Res. 2007 Mar 15; 13(6): 1875-82). La liaison des anticorps aux FcR a été mesurée dans ces études par un dosage de type ELISA (acronyme de « enzyme linked immunosorbent assay »), ou bien de manière fonctionnelle, par la mesure de la réponse ADCC.

De manière générale, une méthode de détection de la liaison d'anticorps potentiellement thérapeutiques aux récepteurs Fc exprimés par une cellule intacte serait un outil précieux pour le développement d'anticorps thérapeutiques, ou encore pour l'étude de mécanismes du système immunitaires médiés par les récepteurs Fc. Les techniques actuellement disponibles pour étudier la liaison d'anticorps aux récepteurs Fc sont relativement fastidieuses.

Le système commercialisé sous le nom Biacore® permet la détection d'interactions entre deux partenaires de liaison et est basé sur le phénomène de la résonnance plasmon de surface. Ce système a été utilisé pour étudier l'effet de la glycosylation de fragments Fc d'anticorps sur leur liaison au récepteur FcγRIII. Il nécessite l'immobilisation du récepteur Fc ou bien d'un fragment du domaine Fc de l'anticorps testé à une micropuce, capable de générer un signal qui dépend de l'indice de réfraction à sa surface (Biacore Journal Number 1, 2009, 15-17). Cette approche présente plusieurs inconvénients, notamment une étape critique de fixation d'un des partenaires de liaison à la surface de la micropuce, la nécessité d'un équipement couteux qui requiert une certaine expertise, et le fait que la technique ne permette pas de travailler avec des cellules vivantes exprimant le récepteur Fc étudié. Par ailleurs, cette approche ne fournit pas non plus d'information quant à l'éventuelle liaison de l'anticorps testé à son antigène dans sa conformation cellulaire.

La demande de brevet Français FR2844521 décrit une approche selon laquelle un anticorps est mis en contact avec des cellules exprimant le récepteur CD16 en présence de l'antigène dudit anticorps, et au moins une cytokine produite par les cellules est mesurée, une augmentation de la quantité de cytokine étant représentative de l'activation desdites cellules. Cette technique ne permet pas la mesure directe de la liaison de l'anticorps au FcR. Elle nécessite également une étape de centrifugation du surnageant pour détecter les cytokines sécrétées par les cellules.

La demande de brevet Européen EP 1 298 219 décrit une méthode de dosage de l'effet du fragment Fc d'un anticorps sur une cellule exprimant un FcR, consistant à immobiliser ledit anticorps sur un support solide, à cultiver des cellules exprimant ledit récepteur en présence dudit anticorps, et à mesurer l'effet éventuel du FcR en présence de l'anticorps. Dans cette méthode, l'activation du FcR est également mesurée de manière indirecte en détectant la production de cytokines par la cellule. Cette technique présente les mêmes inconvénients que celle décrite dans la demande FR2844521, notamment une étape de centrifugation.

La demande de brevet américain US 2009/0176220 présente une variante des approches précédentes consistant à mettre en contact des cellules exprimant des FcR avec des anticorps ayant fait l'objet d'une agrégation préalable. Comme dans les techniques précédentes, l'activation des cellules est déterminée par la mesure de cytokines sécrétées par la cellule.

La société Cisbio Bioassays commercialise une gamme de produit sous la dénomination Tag-lite®, permettant le marquage de protéines exprimées par des cellules par des composés fluorescents, ainsi que des composés fluorescents partenaires de FRET (HTRF®). Le phénomène de FRET entre un composé donneur d'énergie et un composé accepteur étant dépendant de la distance entre ces deux composés, les produits de la gamme Tag-lite® permettent d'étudier des interactions moléculaires dans un contexte cellulaire. Une des applications de cette technique est l'étude de l'interaction entre un récepteur couplé aux protéines G (RCPG) marqué par un partenaire de FRET, avec son ligand marqué par un deuxième partenaire de FRET. Cette approche permet par exemple d'effectuer des dosages par compétition pour évaluer la liaison de nouveaux ligands potentiels de ces RCPG. Un autre exemple d'application de ces produits est l'étude de la dimérisation de RCPG marqués par des composés partenaires de FRET.

Il n'existe pas aujourd'hui de méthode permettant de mesurer directement la liaison d'un anticorps à un récepteur Fc exprimé à la surface d'une cellule intacte. Les techniques existantes présentent plusieurs inconvénients : dosages indirects de cytokines sécrétées par les cellules, étapes de centrifugation peu adaptées à une mise en oeuvre automatisée, ou encore utilisation d'approches nécessitant des étapes préliminaires fastidieuses et dont la mise en oeuvre est relativement longue. Ces techniques ne permettent pas non plus d'obtenir facilement et rapidement des informations sur le niveau de glycosylation d'un anticorps donné et son effet sur la liaison aux récepteurs Fc.

Les inventeurs ont mis au point une méthode permettant de résoudre ces problèmes.

### Description

La demande décrit un procédé *in vitro* de détermination de la liaison d'un anticorps avec un récepteur Fc exprimé dans des membranes cellulaires ou des cellules intactes présentes dans un milieu de mesure, comprenant les étapes suivantes :
(i) Marquage direct ou indirect dudit récepteur Fc par le premier membre d'un couple de partenaires de FRET, ou bien introduction dans le milieu de membranes cellulaires ou de cellules intactes dont les récepteurs Fc ont été préalablement marqués de manière directe par le premier membre d'un couple de partenaires de FRET ;
(ii) Ajout dans le milieu de mesure dudit anticorps, marqué de manière directe ou indirecte par le second membre dudit couple de partenaires de FRET ;
(iii)Mesure du signal de FRET, l'existence d'un signal de FRET étant représentative de la liaison de l'anticorps au récepteur Fc.

Ce procédé peut être notamment utilisé pour déterminer la constante de dissociation d'un complexe récepteur Fc-Anticorps. Dans ce cas, elle est répétée avec différentes concentrations d'anticorps, en particulier des concentrations croissantes d'anticorps, afin d'établir une courbe de saturation, représentant l'évolution du signal de FRET en fonction de la concentration en anticorps. Cette courbe permet de déterminer la constante de dissociation (« Kd »), correspondant à la concentration d'anticorps à 50% de la saturation du récepteur Fc.

L'invention concerne en un format de dosage par compétition. Ainsi, l'invention consiste en un procédé in vitro de détermination de la liaison d'un anticorps (ci-après « anticorps compétiteur ») avec un récepteur Fc exprimé dans des membranes cellulaires ou des cellules intactes présentes dans un milieu de mesure, par compétition avec un anticorps de référence, comprenant les étapes suivantes :
(i) Marquage direct dudit récepteur Fc par le premier membre d'un couple de partenaires de FRET, ou bien introduction dans le milieu de membranes cellulaires ou de cellules intactes dont les récepteurs Fc ont été préalablement marqués de manière directe par le premier membre d'un couple de partenaires de FRET ;
(ii) Ajout dans le milieu de mesure de l'anticorps compétiteur ;
(iii) Ajout dans le milieu de mesure d'un anticorps de référence, marqué de manière directe par le second membre dudit couple de partenaires de FRET ;
(iv) Mesure du signal de FRET, une diminution du signal mesuré en présence de l'anticorps compétiteur par rapport à celui mesuré en son absence étant représentative de la liaison de cet anticorps au récepteur Fc ;
caractérisé en ce que le récepteur Fc est marqué de manière directe via une enzyme suicide, à savoir qu'il est exprimé sous forme d'une protéine de fusion comprenant le récepteur Fc et l'enzyme suicide, et que le marquage est effectué par ajout au milieu de mesure du substrat de l'enzyme, conjugué à un des partenaires de FRET.

Les étapes (i) (ii) et (iii) sont mises en oeuvre de préférence dans cet ordre, mais il est possible de mettre en contact les différents éléments dans un ordre différent (par exemple ((i), (iii), (ii)), ou encore simultanément.

L'étape (iv) est mise en oeuvre après un temps d'incubation qui peut aller de quelques minutes (par exemple 3, 4, 5 minutes), à plusieurs heures (par exemple de 1 à 20 heures).

Si la concentration de l'anticorps compétiteur est connue, alors on peut comparer le signal obtenu à l'étape (iv) avec le signal mesuré lorsque le procédé est mis en oeuvre avec un anticorps de référence non-marqué à la place de l'anticorps compétiteur, et conclure quant à l'affinité de ce dernier pour le récepteur Fc. En particulier, à concentration égale, si le signal de FRET mesuré en présence de l'anticorps compétiteur est inférieur à celui mesuré en présence de l'anticorps de référence non-marqué, alors l'anticorps compétiteur a une meilleure affinité que ce dernier, et inversement. L'anticorps de référence non-marqué peut être le même que celui de l'étape (iii), ou bien un autre anticorps auquel on veut comparer l'anticorps compétiteur.

Par ailleurs, lorsque le procédé de dosage par compétition ci-dessus est reproduit en présence de différentes quantités d'anticorps à tester et d'une quantité fixe d'anticorps de référence les résultats obtenus permettent de déterminer l'EC50 de l'anticorps compétiteur. L'EC50 de l'anticorps peut être comparée à celle de l'anticorps de référence, l'anticorps ayant l'EC50 la plus basse étant celui ayant la meilleure affinité pour le récepteur Fc. Par « EC50 » on entend la concentration permettant d'atteindre 50% d'un effet donné. Ici, l'EC50 d'un anticorps compétiteur correspondra à la concentration de cet anticorps inhibant 50% de la liaison de l'anticorps de référence au récepteur Fc. L'homme du métier est familier de cette notion et des logiciels sont disponibles pour calculer automatiquement les EC50 à partir des résultats obtenus dans le cadre de la mise en oeuvre précédente.

Il est à noter que le procédé selon l'invention ne nécessite pas que l'anticorps compétiteur soit utilisé sous forme purifiée : il peut être inclus dans un mélange, par exemple un surnageant de culture de cellules produisant cet anticorps. C'est l'un des avantages du procédé de l'invention que de pouvoir étudier des anticorps non purifiés, puisqu'il ne nécessite pas d'étapes fastidieuses de préparation des anticorps à tester.

Les procédés ci-dessus sont mis en oeuvre avec des récepteurs Fc exprimés dans des membranes cellulaires ou des cellules intactes. Ces membranes peuvent être préparées de manière classique à partir de cellules soumises à un traitement chimique ou mécanique. Le terme « membranes cellulaires » inclut les membranes de cellules intactes, et la mise en oeuvre de l'invention avec des cellules intactes est particulièrement avantageuse et préférée puisque le procédé est alors effectué dans un contexte relativement proche de la réalité biologique.

Par « anticorps de référence », on entend un anticorps dont on sait de manière certaine qu'il se fixe au récepteur Fc. Ainsi si le récepteur Fc est un récepteur Fc gamma (FcyR), l'anticorps de référence pourra être tout anticorps de la classe des IgG, quelle que soit sa spécificité épitopique. De même, si le récepteur Fc est un récepteur Fc alpha ou epsilon, l'anticorps de référence pourra être tout anticorps de la classe des IgA ou des IgE, respectivement.

De manière inattendue, le procédé selon l'invention est suffisamment sensible pour permettre l'observation de variations d'affinité lorsque certaines modifications sont opérées sur les anticorps. Plus précisément, les inventeurs ont découvert que les modifications connues pour augmenter ou diminuer l'affinité d'un anticorps avec un récepteur Fc étaient corrélées avec des variations d'EC50 mesurées avec le procédé selon l'invention.

Ainsi, dans une mise en oeuvre particulière, l'anticorps de référence et l'anticorps compétiteur diffèrent notamment en ce que ce dernier a été fabriqué selon un procédé, ou bien a fait l'objet d'un traitement, visant à altérer le niveau de glycosylation de son fragment Fc.

En particulier et dans une mise en oeuvre préférée, l'anticorps à tester a été fabriqué selon un procédé, ou bien a fait l'objet d'un traitement, visant à altérer la fucosylation de son fragment Fc. De tels procédés et traitements sont connus de l'homme du métier et sont utilisés pour améliorer l'efficacité de certains anticorps thérapeutiques. Ces traitements, décrits entre autre par Yamane-Ohnuki et al. (MAbs. 2009 May-Jun;1(3):230-6) sont de trois types :
- Procédé de production d'anticorps par génie génétique dans des organismes eucaryotes (levures) ou des cellules végétales dont les voies de N-glycosylation ont été modifiées pour les rendre plus proches de celles des mammifères : cette approche consiste à désactiver certains gènes impliqués dans les mécanismes de N-glycosylation de ces organismes et à en introduire d'autres qui sont spécifiques des voies de N-glycosylation des mammifères.
- Procédé de production d'anticorps par génie génétique dans des cellules de mammifères (1) ayant naturellement une activité intrinsèque de fucosylation α-1,6 limitée, ou (2) dans lesquelles les mécanismes de fucosylation α-1,6 ont été inhibés par l'introduction de petits brins d'ARN interférant (connus sous l'acronyme siRNA), ou encore (3) dans lesquelles ont été introduits l'ADN codant pour la β-1,4-N-acétylglucosaminyltransférase (GnTIII) et celui codant pour l'α-mannosidase II (ManII) du Golgi, ou enfin (4) dont la fonction de fucosylation α-1,6 a été inhibée au niveau du locus génomique responsable de cette voie de glycosylation.
- Contrôle de la fucosylation d'anticorps *in vitro,* par traitement avec des enzymes catalysant la fucosylation de protéines non-fucosylées ou bien des fucosylases capables de cliver les résidus sucrés de protéines fucosylées.

L'invention permet de tester facilement les anticorps ainsi obtenus pour leur capacité à se lier aux récepteurs Fc, ce qui constitue une information très précieuse car indicative de l'efficacité thérapeutique potentielle de ces anticorps.

Une variante de l'invention permet de déterminer le niveau de glycosylation d'un anticorps à tester. Elle est caractérisée en ce qu'elle comprend les étapes suivantes :
- mise en oeuvre du procédé de dosage par compétition avec plusieurs anticorps compétiteurs ayant des niveaux connus de glycosylation ou de déglycosylation, ce procédé étant reproduit en présence de différentes quantités de chaque anticorps compétiteur et d'une quantité fixe d'anticorps de référence puis
- mise en oeuvre du procédé de dosage par compétition mais cette fois avec l'anticorps à tester en tant qu'anticorps compétiteur, ce procédé étant reproduit en présence de différentes quantités d'anticorps à tester et d'une quantité fixe d'anticorps de référence, et
- détermination du niveau de glycosylation de l'anticorps testé par comparaison de son EC50 avec celle de chacun des anticorps compétiteurs dont les niveaux de glycosylation ou de déglycosylation sont connus.

Ce procédé permet en particulier de déterminer le niveau de fucosylation d'un anticorps.

Ces dernières mises en oeuvre permettant l'étude d'anticorps dont la glycosylation ou la fucosylation a été limitée d'une manière ou d'une autre, sont particulièrement adaptées au cas où le récepteur Fc est un récepteur Fc gamma et en particulier le récepteur CD16a ou un de ses variants, dont la liaison aux fragments Fc des anticorps de la classe des IgG1 est connue pour être liée au niveau de fucosylation de ces anticorps. Dans la mesure où le niveau de fucosylation des anticorps est directement corrélé à l'intensité de la réponse de la cellule effectrice, par exemple à l'intensité de la réponse ADCC, ce procédé permet de prédire la capacité de l'anticorps à déclencher une réponse via la cellule effectrice.

Dans les mises en oeuvre précédentes, des membranes cellulaires dont les récepteurs ont été préalablement marqués (et les membranes cellulaires éventuellement congelées) peuvent également être utilisées, et l'étape (i) consiste alors en l'introduction dans le milieu de mesure de membranes cellulaires dont les récepteurs Fc ont été préalablement marqués de manière directe par le premier membre d'un couple de partenaires de FRET. Dans le cas où des cellules marquées congelées sont utilisées, elles auront fait l'objet d'un lavage après leur décongélation et préalablement à leur ajout dans le milieu de mesure.

Les termes utilisés ci-dessus ont les significations suivantes :
« *Couple de partenaires de FRET* » : cette expression désigne un couple constitué d'un composé fluorescent donneur d'énergie (ci-après « composé fluorescent donneur ») et d'un composé accepteur d'énergie (ci-après « composé accepteur »); lorsqu'ils sont à proximité l'un de l'autre et lorsqu'ils sont excités à la longueur d'onde d'excitation du composé fluorescent donneur, ces composés émettent un signal de FRET. Il est connu que pour que deux composés fluorescents soient partenaires de FRET, le spectre d'émission du composé fluorescent donneur doit recouvrir partiellement le spectre d'excitation du composé accepteur.
« *Signal de FRET* » : désigne tout signal mesurable représentatif d'un FRET entre un composé fluorescent donneur et un composé accepteur. Un signal de FRET peut donc être une variation de l'intensité ou de la durée de vie de luminescence du composé fluorescent donneur ou du composé accepteur lorsque ce dernier est fluorescent.
« *Milieu de mesure* » : désigne le contenu du puits d'une plaque, d'un tube à essai ou de tout autre contenant approprié au mélange de cellules ou de membranes cellulaires avec les réactifs nécessaires à la mise en oeuvre de l'invention.

Le récepteur Fc est choisi parmi les récepteurs de la table 1, et de manière préférée est le recepteur CD16a (FcγRIIIa) ou un de ses variants. Plusieurs variants de ce récepteur sont connus, en particulier les variants naturels L66H, L66R, G147D, Y158H, F203S, F176V (ou F158V dans certaines publications). Dans une mise en oeuvre préférée, c'est le variant V158 (ou V176) qui est utilisé. Dans une autre mise en oeuvre c'est le variant F158 (ou F176).

Les différents moyens de marquage du récepteur Fc sont décrits ci-après, mais selon l'invention le récepteur Fc est marqué de manière directe via une enzyme suicide, cette enzyme pouvant être en particulier choisie parmi : les mutants de la déshalogénase, ou un fragment de la protéine de transport d'acyles, les mutants de l'O6-alkylguanine DNA alkyltransférase, ces derniers étant préférés. Comme indiqué par ailleurs, cette méthode de marquage du récepteur Fc implique que les cellules aient fait l'objet d'un traitement préalable permettant l'expression d'une protéine de fusion comprenant le récepteur Fc et l'enzyme suicide. Le marquage sera effectué par ajout du substrat de l'enzyme, conjugué à l'un des membres du couple de partenaires de FRET.

De même, les moyens de marquage de l'anticorps marqué par fluorophore sont décrits ci-après mais de manière préférée, cet anticorps est marqué de manière directe, par liaison covalente avec un des partenaires de FRET.

Dans une mise en oeuvre particulière, le récepteur Fc est marqué par le composé accepteur et l'anticorps par le composé donneur. Dans une autre mise en oeuvre, le récepteur Fc est marqué par le composé donneur et l'anticorps par le composé accepteur. Cette dernière mise en oeuvre est préférée.

### Marauaae du récepteur Fc

### (a) Couplage du récepteur Fc avec un donneur ou un accepteur de manière indirecte (non covalente) - couplage non compris dans la présente invention

Le donneur ou l'accepteur peuvent être couplés avec le récepteur Fc via une protéine capable de s'associer au récepteur Fc, cette protéine étant elle-même marquée de manière directe ou indirecte par le premier membre d'un couple de partenaires de FRET. Il est par exemple connu que le récepteur FcγRIIIA (CD16a) est associé dans la membrane cellulaire à la chaîne gamma du récepteur FcεRI ou à la sous unité zeta de CD3 ; le récepteur CD16a peut ainsi être marqué de manière indirecte via ces chaînes gamma ou zeta, elles-mêmes marquées directement par un composé fluorescent.

Le donneur ou l'accepteur peut être couplé avec le récepteur Fc par l'intermédiaire d'un couple de partenaires de liaison dont l'un au moins est de nature protéique. Dans cette approche, le récepteur Fc est fusionné avec le partenaire de liaison de nature protéique par les techniques classiques de biologie moléculaire (construction d'un vecteur d'expression comprenant une séquence de nucléotides codant pour le récepteur Fc, fusionnée avec celle codant pour le partenaire de liaison protéique, et introduction du vecteur d'expression dans la cellule). Le donneur ou l'accepteur est conjugué de manière covalente à l'autre partenaire de liaison, que l'on appelle ici agent de couplage, qui sera ensuite ajouté au milieu extracellulaire. La reconnaissance des partenaires de liaison permet le marquage indirect du récepteur Fc par le donneur ou l'accepteur.

A titre d'exemple non limitatif de ce type de partenaires de liaison, on peut citer :
- Le couple constitué par un anticorps spécifique d'un épitope naturellement présent sur le récepteur Fc, marqué par un composé fluorescent, et cet épitope. Lorsque cette méthode de marquage est utilisée, il convient de vérifier que la liaison de cet anticorps à cet épitope ne gêne pas la liaison du récepteur Fc aux fragments Fc d'anticorps. Par ailleurs, dans cette mise en oeuvre, il convient d'éviter d'utiliser un anticorps dont le fragment constant pourrait être reconnu par le récepteur Fc d'intérêt.
- Le couple constitué de la séquence cystéine-cystéine-X-X-cystéine-cystéine (SEQ ID N°1) dans laquelle X est un acide aminé quelconque et d'un composé bi-arsenic. Ces composés bi-arsenic peuvent être facilement marqués avec une molécule organique du type fluorescéine ou rhodamine (voir B.A.Griffin et al. (1998) Science. 1998, 281, 269-271 et S.A. Adams et al. (2002) J. Am. Chem. Soc. 2002, 124, 6063-6076 pour des détails sur la technologie).
- Le peptide BTX (bungarotoxine), composé de 13 acides aminés qui est reconnu par la bungarotoxine (BTX), peut être couplé à une molécule fluorescente (voir C. M. McCann et al. (2005), Biotechnique (2005), 38, 945-952).
- Le couple streptavidine (ou avidine) / biotine : la séquence de liaison de la Streptavidine (SBP-Tag) est une séquence formée par 38 acides aminés qui présente une haute affinité pour la biotine pouvant être préalablement marquée avec un donneur ou un accepteur (voir C. M. McCann et al. (2005), Biotechnique (2005), 38, 945-952).
- La séquence de l'enzyme dihydrofolate réductase d'E. coli (eDHFR) qui lie spécifiquement et avec une haute affinité des ligands, tels que la triméthoprime sur lesquels peuvent être greffés le donneur ou l'accepteur selon la technologie dénommée « Ligand link Universal labelling technology » de la Société Active Motif.
- Les couples tags/antitags sont des partenaires de liaison fréquemment utilisés pour marquer des protéines. Le terme « tag » désigne une petite « étiquette » protéique constituée d'une séquence d'acides aminés, généralement mais pas obligatoirement assez courte (moins de 15 acides aminés), qui est fusionnée au récepteur Fc. Le terme « antitag » désigne un anticorps se liant de manière spécifique audit « tag ». Dans cette mise en oeuvre, l'anticorps « antitag » est lié de manière covalente au donneur ou à l'accepteur. Lorsque l'anticorps ainsi marqué est ajouté au milieu extracellulaire, il se lie au « tag » conjugué au récepteur Fc et l'interaction « tag/antitag » permet le marquage indirect de cette protéine par le donneur ou l'accepteur. A titre d'exemple non limitatif de couples « tags/antitags », on peut citer les couples suivants dont les membres sont disponibles commercialement : GST/anticorps anti-GST dans lequel GST représente la glutathione S-transférase ou un de ses fragments ; 6HIS/anticorps anti-6HIS dans lequel 6HIS est un peptide constitué de 6 histidines ; Myc/anticorps anti-Myc dans lequel Myc est un peptide constitué des acides aminés 410-419 de la protéine Myc humaine ; FLAG/anticorps anti-FLAG dans lequel FLAG est un peptide ayant les 8 acides aminés DYKDDDDK (SEQ ID N°2) ; HA/anticorps anti HA dans lequel HA est un épitope de l'hémaglutinine d'Influenza, constitué des 9 acides aminés YPYDVPFYA (SEQ ID N°3). Il est clair que la nature exacte du tag n'est pas critique pour la mise en oeuvre du procédé.

### (b) Couplage du récepteur Fc avec un donneur ou un accepteur de manière covalente - couplage non compris dans la présente invention

Dans cette approche, le donneur ou l'accepteur est couplé au récepteur Fc par une liaison covalente ; plusieurs techniques ont été décrites et les réactifs nécessaires à leurs mises en oeuvre sont disponibles commercialement. Pour ce couplage, on pourra utiliser l'une des techniques ci-après :
- Formation d'une liaison covalente au niveau d'un groupe réactif présent sur le récepteur Fc, en particulier au niveau d'un des groupes suivants : le groupe amino terminal, les groupes carboxylates des acides aspartiques et glutamiques, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.
   Ces groupes présents sur le récepteur Fc peuvent former une liaison covalente avec un groupe réactif porté par le donneur ou l'accepteur. Les groupes réactifs appropriés sont connus de l'homme du métier : un donneur ou l'accepteur fonctionnalisé par un groupe maléimide sera par exemple capable de se lier de manière covalente avec les groupes thiols portés par les cystéines de la protéine. De même, un donneur/accepteur portant un ester de N-hydroxysuccinimide sera capable de se fixer de manière covalente à une amine du récepteur membranaire.

### (c) Couplage du récepteur Fc avec un donneur ou un accepteur de manière covalente en utilisant une enzyme suicide- couplage selon l'invention

Par enzyme suicide on entend des protéines qui ont une activité enzymatique modifiée par des mutations spécifiques qui leur confèrent la capacité de lier rapidement et de façon covalente un substrat. Ces enzymes sont dites « suicides » car chacune ne peut lier qu'une seule molécule fluorescente, l'activité de l'enzyme étant bloquée par la fixation du substrat. Ces enzymes constituent par conséquent un outil de choix pour marquer spécifiquement des récepteurs d'intérêt avec un rapport d'une molécule fluorescente pour une protéine. Dans cette approche, une enzyme suicide est fusionnée, par les techniques classiques de biologie moléculaire, avec le récepteur Fc -de préférence dans sa partie N-terminale- et le substrat de l'enzyme lié de manière covalente à un donneur/accepteur est introduit dans le milieu extracellulaire. La réaction enzymatique a pour conséquence la liaison covalente du substrat marqué à l'enzyme, et donc le marquage du récepteur Fc par le donneur ou l'accepteur.

On peut citer à titre d'exemple non limitatif les enzymes suivantes :
- les mutants de l'O6-alkylguanine DNA alkyltransférase (AGT). Les enzymes SNAP-tag (Juillerat et al, Chemistry & biology, Vol.10, 313-317 Avril 2003) et CLIP-tag (Gautier et al, Chemistry et Biology, 15, 128-136, février 2008) commercialisées par la société Cisbio Bioassays sont des mutants de l'AGT humaine dont les substrats sont respectivement, l'O⁶-benzylguanine (ci-après abrégée BG) et l'O²-benzylcytosine (ci-après abrégée BC). L'enzyme N-AGT (Gronemeyer et al. (Protein engineering, design & selection, vol. 19, no 7, pp 309-3016, 2006)) est un autre mutant de cette enzyme dont la réactivité avec l'O⁶-benzylguanine est meilleure que celle de l'enzyme SNAP-tag.
- les mutants d'une déshalogénase (telle que l'enzyme HaloTag commercialisée par Promega) qui génère également une réaction enzymatique du type suicide (voir WO2004/072232), dont certains des substrats sont des composés de la famille des chloroalcanes, en particulier les chloroalcanes comportant le motif -NH-CH₂CH₂-O-CH₂CH₂-O-(CH₂)₆-Cl. Dans ce cas, le donneur/accepteur sera conjugué à ce type de motif.
- La protéine ACP (« Acyl Carrier Protein »), protéine transporteur d'acyles, sur laquelle est transféré, en présence de phosphopantéthéine transférase, le résidu 4'-phosphopantéthéine du coenzyme A sur une sérine de l'ACP (N. George et al, Journal of the American Chemical society 126 (2004) p 8896-8897). Lorsque cette approche est utilisée pour marquer le récepteur Fc par le donneur ou l'accepteur, il est nécessaire de rajouter au milieu réactionnel de la phosphopantéthéine transférase. La société NEB commercialise un fragment de l'ACP sous le nom commercial « ACP-Tag » pour le marquage de protéines.

Lorsque cette approche est utilisée pour marquer le récepteur d'intérêt, les cellules sont transfectées avec un plasmide d'expression comportant l'ADN codant pour une protéine de fusion comprenant l'enzyme suicide et le récepteur d'intérêt. Ce plasmide peut également comprendre, en amont de l'ADN codant pour ces protéines, l'ADN codant pour une étiquette telle que par exemple l'épitope FLAG, l'épitope myc, ou encore celui de l'hémaglutinine de l'influenza (HA). Ces étiquettes ne sont pas essentielles mais facilitent la manipulation de la protéine de fusion à des fins de contrôle ou de purification. La transfection est opérée par les techniques classiques, telle que l'électroportion.

Pour s'assurer que la protéine de fusion sera exprimée dans la membrane cellulaire, il peut être utile d'inclure dans le plasmide d'expression, en amont de la séquence codant pour le récepteur d'intérêt et de l'enzyme suicide, celle codant pour un peptide d'adressage membranaire, tels que le peptide signal T8 ou le peptide signal du récepteur mGluR5, dont l'utilisation à cet effet est connue de l'homme du métier. Enfin, il peut également être souhaitable de s'assurer que la séquence codant pour le récepteur d'intérêt ne comporte pas de séquence d'adressage membranaire native qui pourrait faire l'objet d'un clivage post-traductionnel de la liaison entre le récepteur d'intérêt et l'enzyme suicide : si c'est le cas, il est préférable de ne pas introduire ce domaine dans le plasmide d'expression.

Finalement, lorsqu' une enzyme suicide est utilisée pour marquer le récepteur Fc avec le partenaire de FRET, l'invention comprend une étape préliminaire de transfection des cellules par un vecteur d'expression comprenant la séquence d'ADN codant pour une protéine de fusion correspondant au récepteur Fc, fusionné dans sa partie N-terminale avec une enzyme suicide. Les techniques de transfection telles que l'électroporation ou l'utilisation de lipofectamine sont connues de l'homme du métier.

L'introduction dans le milieu extracellulaire du substrat de l'enzyme conjugué à un partenaire de FRET aura pour conséquence le marquage du récepteur d'intérêt par ce partenaire de FRET.

La société Cisbio Bioassays commercialise des plasmides Tag-Lite® permettant l'expression de protéines de fusion avec les enzymes suicides connues sous les noms commerciaux de SNAP-tag®, CLIP-tag® et Halotag®. Les séquences d'ADN codant pour les récepteurs Fc de la table 1 sont connues et sont disponible dans les bases de données telles que Genbank.

### Marquage de l'anticorps

L'anticorps peut également être marqué de manière directe ou indirecte. Selon l'invention, l'anticorps est marqué de manière directe.

Le marquage direct de l'anticorps par un composé fluorescent peut être effectué par les méthodes classiques connues de l'homme du métier, reposant sur la présence de groupes réactifs sur l'anticorps, en particulier des groupes suivants : le groupe amino terminal, les groupes carboxylates des acides aspartiques et glutamiques, les groupes amines des lysines, les groupes guanidines des arginines, les groupes thiols des cystéines, les groupes phénols des tyrosines, les cycles indoles des tryptophanes, les groupes thioéthers des méthionines, les groupes imidazoles des histidines.

Ces groupes peuvent former une liaison covalente avec un groupe réactif porté par le composé fluorescent Les groupes réactifs appropriés sont connus de l'homme du métier : un donneur ou l'accepteur fonctionnalisé par un groupe maléimide sera par exemple capable de se lier de manière covalente avec les groupes thiols portés par les cystéines de la protéine. De même, un donneur/accepteur portant un ester de N-hydroxysuccinimide sera capable de se fixer de manière covalente à une amine présente sur l'anticorps.

Sans être compris dans la présente invention, l'anticorps peut également être marqué par un composé fluorescent de manière indirecte, par exemple en introduisant dans le milieu de mesure un autre anticorps, lui-même lié de manière covalente à un composé fluorescent, ce deuxième anticorps reconnaissant de manière spécifique le premier anticorps. Lorsque cette approche est utilisée, il convient de choisir un anticorps secondaire dont le domaine Fc ne sera pas reconnu par le récepteur Fc.

Sans être compris dans la présente invention, un autre moyen de marquage indirect très classique consiste à fixer de la biotine sur l'anticorps à marquer, puis d'incuber cet anticorps biotinylé en présence de streptavidine marquée par un fluorophore. Des anticorps biotinylés sont disponibles dans le commerce et la société Cisbio Bioassays commercialise par exemple de la streptavidine marquée avec le fluorophore dont la dénomination commerciale est « d2 » (ref 610SADLA).

Dans la mesure où l'anticorps dont on veut mesurer la liaison au récepteur Fc est lié à ce récepteur via son domaine Fc, ses domaines variables restent disponibles pour reconnaitre l'antigène dont ils sont spécifiques. Sans que être compris dans la présente invention, il est donc également possible de marquer l'anticorps de manière indirecte en introduisant dans le milieu de mesure l'antigène de cet anticorps, cet antigène étant marqué par un composé fluorescent.

### Couples de partenaires de FRET

Selon l'invention, les récepteurs Fc et au moins un des anticorps reconnus par ces récepteurs sont chacun marqués par un membre d'un couple de partenaires de FRET, en particulier par un composé fluorescent donneur ou un composé fluorescent accepteur d'énergie.

Le FRET est défini comme un transfert d'énergie non radiatif résultant d'une interaction dipôle-dipôle entre un donneur et un accepteur d'énergie. Ce phénomène physique nécessite une compatibilité énergétique entre ces molécules. Cela signifie que le spectre d'émission du donneur doit recouvrir, au moins partiellement, le spectre d'absorption de l'accepteur. En accord avec la théorie de Förster, le FRET est un processus qui dépend de la distance séparant les deux molécules, donneur et accepteur : lorsque ces molécules sont à proximité l'une de l'autre, un signal de FRET sera émis.

La sélection du couple de fluorophores donneur / accepteur pour obtenir un signal de FRET est à la portée de l'homme du métier. Des couples donneur-accepteur utilisables pour étudier les phénomènes de FRET sont notamment décrits dans l'ouvrage de Joseph R. Lakowicz (Principles of fluorescence spectroscopy, 2nd edition 338), auquel l'homme du métier pourra se référer.

Les composés fluorescents donneurs d'énergie à durée de vie longue (> 0,1 ms, de préférence comprise entre 0,5 et 6 ms), en particulier les chélates ou cryptates de terres rares sont avantageux puisqu'ils permettent d'effectuer des mesures en temps résolu, c'est-à-dire de mesurer des signaux de TR-FRET (en langue anglaise, « Time Resolved FRET ») en s'affranchissant d'une grande partie du bruit de fond émis par le milieu de mesure. Ils sont pour cette raison et de manière générale préférés pour la mise en oeuvre du procédé selon l'invention. Avantageusement, ces composés sont des complexes de lanthanides. Ces complexes (tels que chélates ou cryptates) sont particulièrement appropriés en tant que membre du couple de FRET donneur d'énergie.

Les complexes de dysprosium (Dy3+), de samarium (Sm3+), de neodymium (Nd3+), d'ytterbium (Yb3+) ou encore d'erbium (Er3+) sont des complexes de terres rares également appropriés aux fins de l'invention, mais les complexes d'europium (Eu3+) et de terbium (Tb3+) sont particulièrement préférés.

De très nombreux complexes de terres rares ont été décrits et plusieurs sont actuellement commercialisés par les sociétés PerkinElmer, Invitrogen et Cisbio Bioassays.

Des exemples de chélates ou cryptates de terres rares appropriés aux fins de l'invention sont :
- Les cryptates de lanthanides, comportant un ou plusieurs motifs pyridine. De tels cryptates de terre rare sont décrits par exemple dans les brevets EP 0 180 492, EP 0 321 353, EP 0 601 113 et dans la demande internationale WO 01/96 877. Les cryptates de terbium (Tb3+) et d'europium (Eu3+) sont particulièrement appropriés aux fins de la présente invention. Des cryptates de lanthanides sont commercialisés par la société Cisbio Bioassays. On peut citer à titre d'exemple non limitatif les cryptates d'europium de formules ci-dessous (qui peuvent être couplés au composé à marquer via un groupe réactif, ici par exemple un groupe NH₂) : 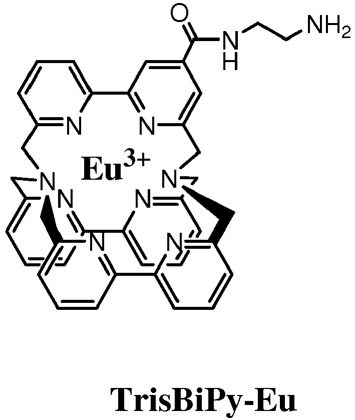 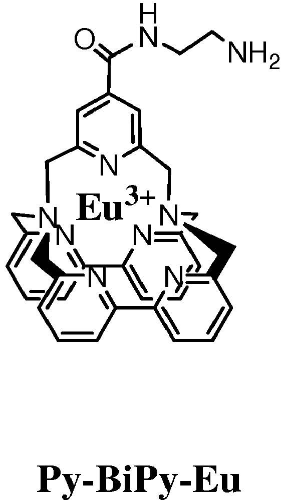 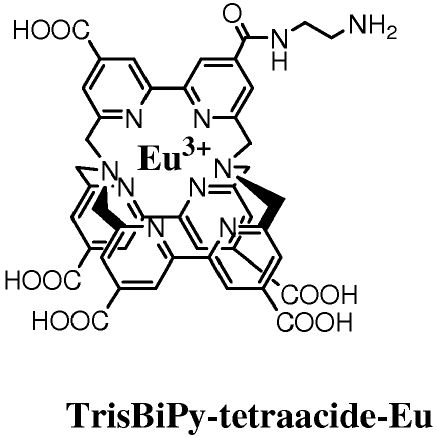 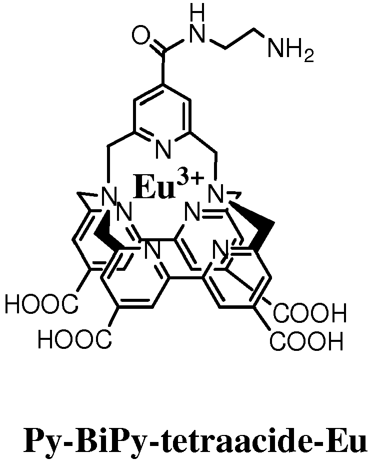
- Les chélates de lanthanides décrits notamment dans les brevets US 4 761 481, US 5 032 677, US 5 055 578, US 5 106 957, US 5 116 989, US 4 761 481, US 4 801 722, US 4 794 191, US 4 637 988, US 4 670 572, US 4 837 169, US 4 859 777. Les brevets EP 0 403 593, US 5 324 825, US 5 202 423, US 5 316 909 décrivent des chélates composés d'un ligand nonadenté tel que la terpyridine. Des chélates de lanthanides sont commercialisés par la société PerkinElmer.
- Des complexes de lanthanides constitués d'un agent chélatant, tel que le tétraazacyclododécane, substitué par un chromophore comportant des cycles aromatiques, tels que ceux décrits par Poole R. et al. dans Biomol. Chem, 2005, 3, 1013-1024 "Synthesis and characterisation of highly emissive and kinetically stable lanthanide complexes suitable for usage in cellulo", peuvent également être utilisés. On peut aussi utiliser les complexes décrits dans la demande WO 2009/10580.
- Les cryptates de lanthanides décrits dans les brevets EP 1 154 991 et EP 1 154 990 sont également utilisables.
- le cryptate de terbium de formule ci-dessous (qui peut être couplé à un composé à marquer via un groupe réactif, ici par exemple un groupe NH₂) : 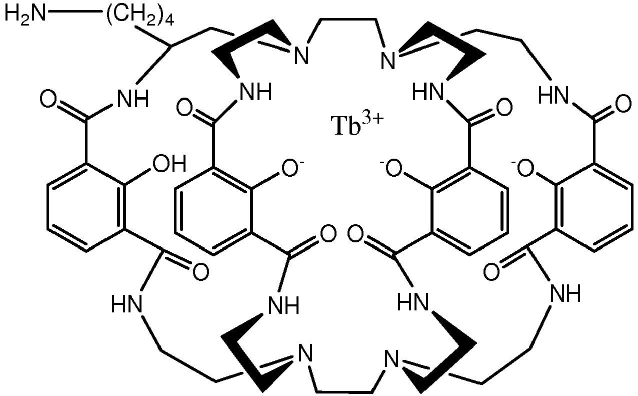 et dont la synthèse est décrite dans la demande internationale WO 2008/063721 (composé 6a page 89).
- Le cryptate de terbium Lumi4-Tb de la société Lumiphore, commercialisé par Cisbio Bioassays.
- Le quantum dye de la société Research Organics, de formule ci-dessous (qui peut être couplé au composé à marquer via un groupe réactif, ici NCS) : 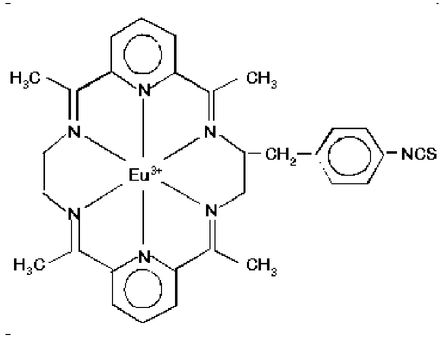
- Les chélates de ruthénium, en particulier les complexes constitués d'un ion ruthénium et de plusieurs bipyridines comme le ruthénium(II) tris(2,2'-bipyridine).
- Le chélate de terbium DTPA-cs124 Tb, commercialisé par la société Life technologies de formule ci-dessous (qui peut être couplé au composé à marquer via un groupe réactif R) et dont la synthèse est décrite dans le brevet américain US 5 622 821. 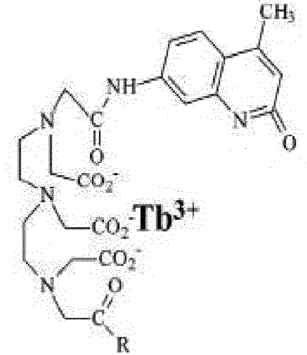
- Le chélate de terbium de formule ci-dessous et décrit par Latva et al (Journal of Luminescence 75: 149-169) :

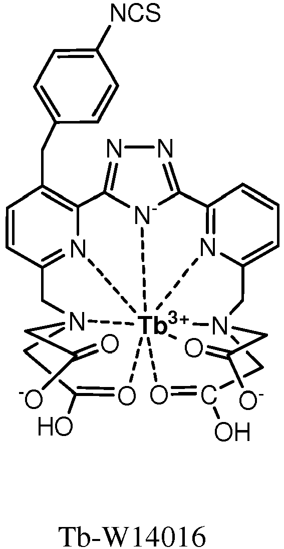

De manière particulièrement avantageuse, le composé fluorescent donneur est choisi parmi : un cryptate d'europium; un chélate d'europium ; un chélate de terbium ; un cryptate de terbium ; un chélate de ruthénium ; et un quantum dye ; les chélates et les cryptates d'europium et de terbium étant particulièrement préférés.

Les complexes de dysprosium (Dy3+), de samarium (Sm3+), de néodymium (Nd3+), d'ytterbium (Yb3+) ou encore d'erbium (Er3+) sont également des complexes de terres rares appropriés aux fins de l'invention.

Les composés fluorescents accepteurs peuvent être choisis dans le groupe suivant : les allophycocyanines, en particulier celles connues sous la dénomination commerciale XL665 ; les molécules organiques luminescentes, telles que les rhodamines, les cyanines (comme par exemple Cy5), les squaraines, les coumarines, les proflavines, les acridines, les fluorescéines, les dérivés du boron-dipyrrométhène (commercialisés sous la dénomination « Bodipy »), les fluorophores connus sous la dénomination « Atto », les fluorophores connus sous la dénomination «DY», les composés connus sous la dénomination « Alexa », le nitrobenzoxadiazole. Avantageusement, les composés fluorescents accepteurs sont choisis parmi les allophycocyanines, les rhodamines, les cyanines, les squaraines, les coumarines, les proflavines, les acridines, les fluorescéines, les dérivés du boron-dipyrrométhène, le nitrobenzoxadiazole.

Les expressions « les cyanines » et « les rhodamines » doivent être respectivement comprises comme « les dérivés de cyanine » et « les dérivés de rhodamine ». L'homme du métier connaît ces différents fluorophores, disponibles dans le commerce.

Les composés « Alexa » sont commercialisés par la société Invitrogen; les composés « Atto » sont commercialisés par la société Attotec ; les composés « »DY» » sont commercialisés par la société Dyomics ; les composés « Cy » sont commercialisés par la société Amersham Biosciences ; les autres composés sont commercialisés par divers fournisseurs de réactifs chimiques, tels que les sociétés Sigma, Aldrich ou Acros.

Les protéines fluorescentes suivantes peuvent également être utilisées en tant que composé fluorescent accepteur : les protéines fluorescentes cyans (AmCyanl, Midori-Ishi Cyan, mTFP1), les protéines fluorescentes vertes (EGFP, AcGFP, TurboGFP, Emerald, Azami Green, ZsGreen), les protéines fluorescentes jaunes (EYFP, Topaz, Venus, mCitrine, YPet, PhiYFP, ZsYellowl, mBanana), les protéines fluorescentes oranges et rouges (Orange kusibari, mOrange, tdtomato, DsRed, DsRed2, DsRed-Express, DsRed-Monomer, mTangerine, AsRed2, mRFP1, JRed, mCherry, mStrawberry, HcRed1, mRaspberry, HcRed-Tandem, mPlim, AQ143), les protéines fluorescentes dans le rouge lointain (mKate, mKate2, tdKatushka2).

Aux fins de l'invention, les dérivés de cyanines ou de la fluoréscéine sont préférés comme composés fluorescents accepteurs.

### Nécessaire de réactifs, cellules

La description concerne également des vecteurs d'expression, « kits » ou nécessaires de réactifs pour la mise en oeuvre des procédés selon l'invention.

Les vecteurs d'expression selon la description sont des plasmides permettant l'expression d'une protéine de fusion comprenant la séquence d'ADN codant pour le récepteur Fc d'intérêt et la séquence codant pour une enzyme suicide, en particulier choisie parmi les mutants de la déshalogénase, un fragment de la protéine de transport d'acyles ou les mutants de l'O6-alkylguanine DNA alkyltransférase, ces derniers étant préférés. Ils peuvent être obtenus en intégrant la séquence d'ADN codant pour le récepteur Fc d'intérêt dans un des plasmides commercialisé par la société Cisbio Bioassays sous les dénominations Tag-lite® SNAP-tag®, CLIP-tag® et Halo-tag®.

La description concerne aussi des cellules, notamment des cellules de mammifères, qui ont été transfectées de manière stable ou transitoire avec les vecteurs d'expression selon l'invention. Les techniques d'introduction de vecteurs d'expression dans les cellules, telle que l'életroporation ou encore l'utilisation de lipofectamine, sont connues de l'homme du métier. Dans un aspect particulièrement avantageux, ces cellules ont été incubées en présence du substrat de l'enzyme suicide, conjugué à un membre d'un couple de partenaires de FRET, et le récepteur Fc qu'elles expriment est ainsi marqué. Ces cellules peuvent être conditionnées sous forme congelée pour faciliter leur conservation et leur distribution aux utilisateurs.

Les nécessaires ou trousses de réactifs selon l'invention comprennent les vecteurs d'expression ou les cellules ci-dessus, accompagnés d'un ou des deux membres d'un couple de partenaires de FRET. Ces partenaires de FRET peuvent être conjugués au substrat de l'enzyme suicide, et/ou bien conjugués à un anticorps de référence dont le fragment Fc est capable de se lier aux récepteurs Fc exprimés par les cellules.

De manière préférée, dans les plasmides et nécessaires de réactifs précédents, le récepteur d'intérêt est un récepteur Fc gamma est en particulier le récepteur CD16a ou l'un de ses variants.

### EXEMPLES

### Exemple 1 : Matériel et méthode

### Réactifs utilisés :

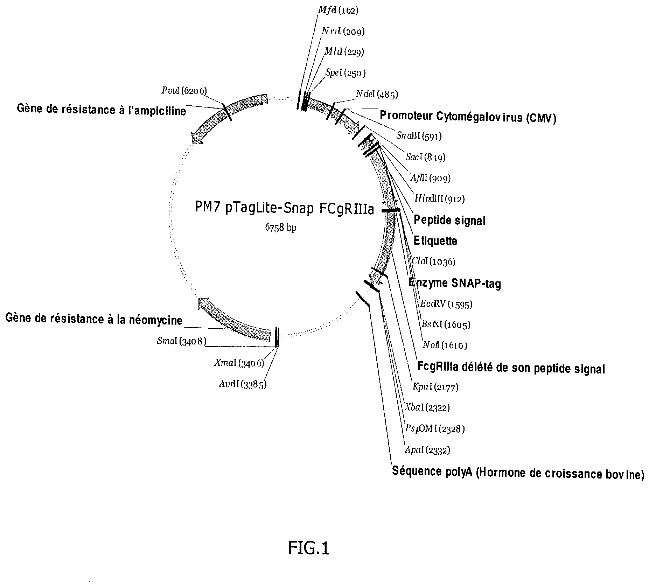

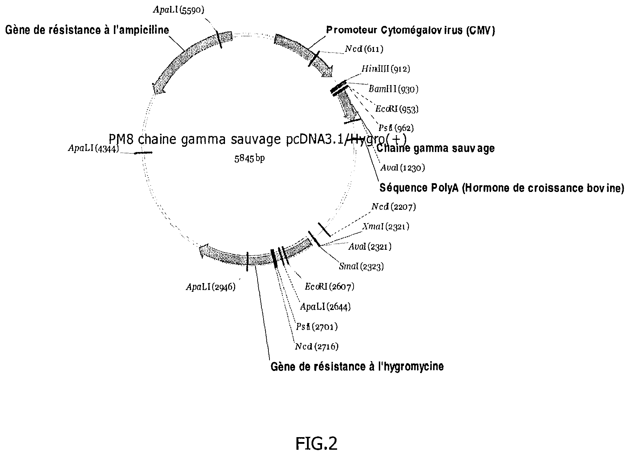

*Milieu de culture* : DMEM/Glutamax + 10 % SVF+ streptomycine (50µg/ml), pénicilline 50 U/ml, HEPES 2mM, acides aminés non essentiels 1% (InVitrogen) OptiMEM : milieu de culture utilisé pour la transfection, Invitrogen
*Lipofectamine 2000:* Invitrogen
*Plasmide SNAP-CD16A* : préparé en insérant la séquence nucléique (SEQ ID N°4) codant pour le récepteur CD16A (variant V158 (ou V176) ref. Genbank NM_000569.6, séquence protéique NP_000560.5) dans le plasmide SNAP-tag® pT8-SNAP-Neomycin (Cisbio Bioassays), voir figure 1.
*Plasmide chaîne gamma du récepteur FcεRI* : La séquence d'ADN (SEQ ID N°5) codant pour la chaine gamma du récepteur FcεRI a été insérée dans un plasmide d'expression par les techniques classiques. La carte de ce plasmide est représentée à la figure 2. Il est connu que le récepteur CD16a forme des dimères avec cette chaîne gamma, et il est donc conseillé de coexprimer les deux protéines. Les inventeurs ont néanmoins découvert que cette co-expression n'était pas nécessaire pour mettre en oeuvre le procédé selon l'invention.
*SVF* : sérum de veau fétal, Invitrogen
*DMSO* : Diméthylsulfoxide, SIGMA
*Tag-lite*® *SNAP-Lumi4Tb* : Composé donneur, Cisbio Bioassays, ref SSNPTBX
*Tag-lite*® : tampon de marquage, Cisbio Bioassays, ref LABMED.

### Transfection des cellules :

Un mélange de transfection contenant 5 µl de plasmide « SNAP-CD16A » (1µg/µl), 15 µl de Lipofectamine et 2,6 ml de milieu OptiMEM et un mélange de 10 µl de plasmide « chaîne gamma » (1µg/µl), 30 µl de Lipofectamine et 5,4 ml de milieu OptiMEM ont été incubés pendant 20 minutes à température ambiante.
Des cellules HEK293 ont été mises en culture dans une flasque T175. Une fois que ces cellules ont atteint une confluence de 60 à 70%, le milieu de culture a été retiré et les cellules ont été lavées avec 10 ml de milieu PBS. 8 ml du mélange de transfection ont ensuite été ajoutés à ces cellules, ainsi que 12 ml de milieu de culture. Les cellules ont ensuite été incubées pendant 1 nuit à 37°C.

### Marquage avec le composé fluorescent donneur Lumi4® Tb

Après retrait du mélange de transfection et lavage avec 10 ml de PBS, 10 ml de composé fluorescent donneur Tag-lite® SNAP-Lumi4-Tb (100nM) en solution dans le tampon de marquage Tag-lite® , ont été ajoutés.
Après incubation de 1h à 37°C, le mélange a été lavé 4 fois avec le tampon de marquage Tag-lite®, puis on a ajouté 5 ml de tampon « cell dissociation buffer » (Sigma) pour dissocier les cellules et 5 ml de milieu OptiMEM. Les cellules ainsi obtenues ont été centrifugées pendant 5min à 1200 tr/mn. Le culot a ensuite été resuspendu avec 1 ml + 1 ml de tampon de marquage Tag-lite® afin de pouvoir compter les cellules.
Cette suspension a été re-centrifugée 5 min à 1200 tr/mn et le culot a été repris par du milieu de culture contenant du SVF 10% et DMSO 10% pour obtenir une suspension de cellules marquées à la concentration de 1 million de cellules / ml. Cette suspension a été aliquotée dans des tubes à raison de 1 ml par tube, et les tubes ont été placés dans une boite Nalgene à -80°C.

### Mesure des signaux de FRET

Dans les exemples suivants, les signaux de FRET ont été mesurés en temps résolu sur un appareil compatible avec HTRF, le PHERAstarFS (BMG Labtech) avec un délai de 60µs et un temps d'intégration de 400µs.

### Exemple 2

Les cellules préparées selon la méthode décrite dans l'exemple 1 ont été décongelées à 37°C et rapidement mélangées à 15 ml de PBS. La suspension obtenue a été centrifugée 5 min à 1200 tr/mn et le surnageant a été retiré. Le culot a été re-suspendu dans du tampon Tag-lite® pour obtenir une suspension permettant la distribution de cellules HEK-CD16a-Tb dans les puits d'une plaque multipoint 384 LV à la concentration de 10 000 cellules par puits sous 10µl.
Un anticorps humain d'isotype IgG1 (peu importe sa spécificité épitopique) non marqué a été ajouté à différentes concentrations finales de 0,3 nM à 5µM sous 5µl. Le même anticorps, marqué par le fluorophore accepteur d2 (kit de marquage d2 Cisbio Bioassays, ref 62D2DPEA), a été ajouté à 200 nM sous 5µl pour une concentration finale de 50 nM.

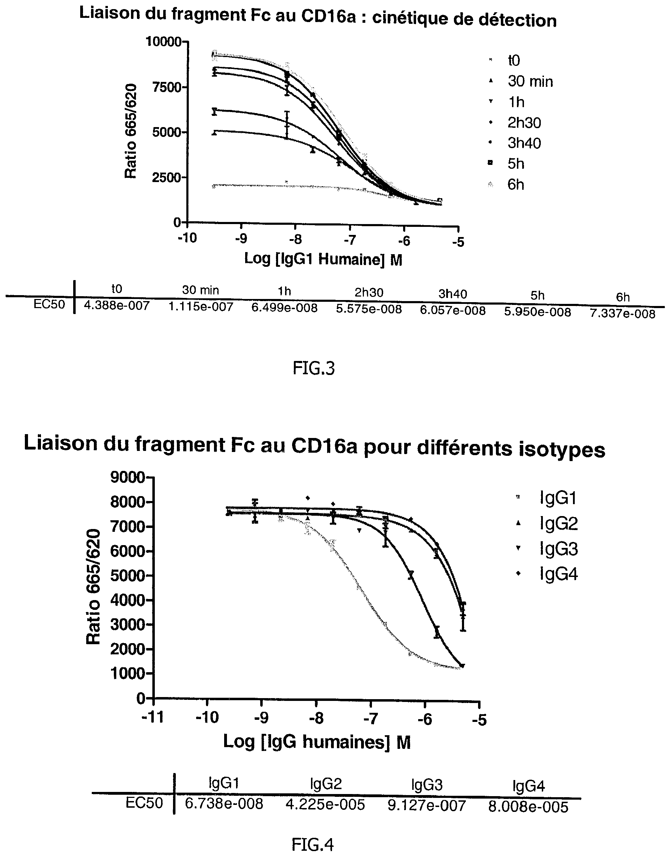

Les signaux de FRET émis par la plaque 384 ont été mesurés immédiatement, puis après 30 min, 1h, 2h30, 3h40, 5h et 6h.
Les résultats présentés sur la figure 3 montrent que le procédé selon l'invention permet de suivre efficacement la cinétique de liaison du fragment Fc d'un anticorps au récepteurs CD16a.

### Exemple 3

L'exemple 2 a été reproduit mais cette fois l'anticorps IgG1 non-marqué (anticorps compétiteur) a été remplacé par des anticorps humains d'isotypes IgG2, IgG3 et IgG4 (peu importe leurs spécificités épitopiques) dont il est connu qu'ils n'ont pas une aussi bonne affinité pour le récepteur CD16a que les anticorps de type IgG1. Après ajout de différentes concentrations de ces anticorps, le milieu réactionnel a été incubé pendant 4h20.
Les résultats présentés dans la figure 4 confirment ce qui était connu en termes d'affinité des IgG2, IgG3 et IgG4 pour le récepteur CD16a et valident donc le procédé selon l'invention, qui permet de comparer efficacement un anticorps de référence avec d'autres anticorps et qui est suffisamment sensible pour permettre de visualiser des différences d'affinité de ces anticorps, ici pour le récepteur CD16a.

### Exemple 4

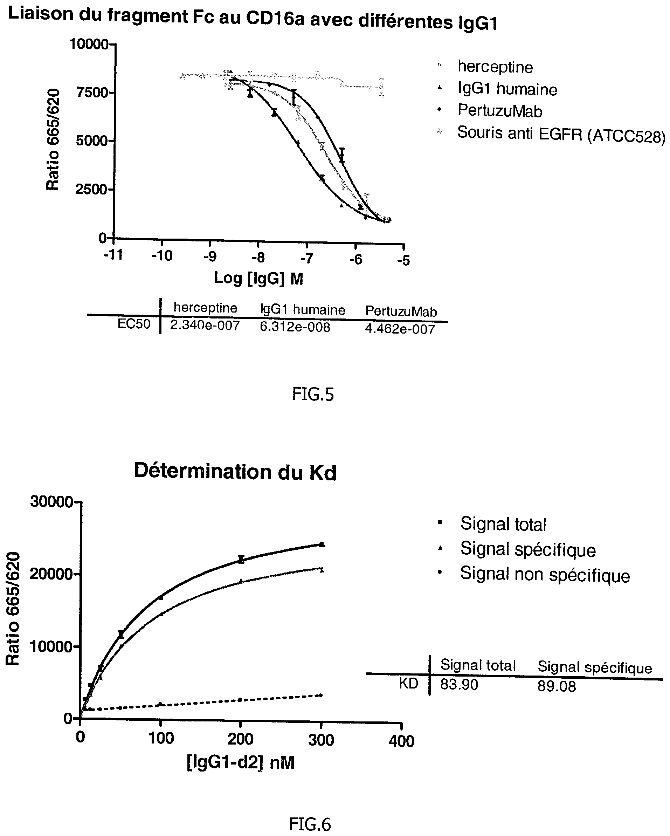

L'exemple 2 a été reproduit mais cette fois l'anticorps IgG1 non-marqué (anticorps compétiteur) a été remplacé par différents anticorps de la même sous-classe : herceptin, pertuzumab et un anticorps de souris anti-EGFR (ATCC528).
Les résultats présentés dans la figure 5 confirment que le procédé selon l'invention mis en oeuvre avec le récepteur CD16a permet de distinguer un anticorps de souris, qui ne se fixe pas au CD16a, d'autres anticorps IgG1 humains, et est suffisamment sensible pour permettre de déterminer des différences d'affinité entre plusieurs anticorps de classe IgG1.

### Exemple 5

Les cellules préparées selon la méthode décrite dans l'exemple 1 ont été décongelées à 37°C et rapidement mélangées à 15 ml de PBS. La suspension obtenue a été centrifugée 5 min à 1200 tr/mn et le surnageant a été retiré. Le culot a été re-suspendu dans du tampon Tag-lite® pour obtenir une suspension permettant la distribution de cellules HEK-CD16a-Tb dans les puits d'une plaque multipoint 384 LV à la concentration de 10 000 cellules par puits sous 10µl.

Un anticorps IgG1 humain marqué a été ajouté dans chaque puits à différentes concentrations pour une concentration finale de 1,5 à 300nM, sous 5µl.
Pour mesurer l'impact de la fixation non spécifique de l'anticorps marqué sur les cellules, l'anticorps IgG1 non marqué a été également ajouté à une concentration finale de 3µM sous 5µl, ce volume étant remplacé par du tampon Tag-lite® pour obtenir le signal global.
Le signal spécifique est obtenu par soustraction du signal non-spécifique au signal global.
Les signaux de FRET émis par la plaque 384 ont été mesurés à différent temps, les résultats après 20h d'incubation sont présentés dans la figure 6.
Cet exemple montre que le procédé selon l'invention permet de déterminer la constante de dissociation du complexe IgG1 - CD16a sur cellules vivantes, et avec un bruit de fond très réduit.

### Exemple 6 : Fucosvlation

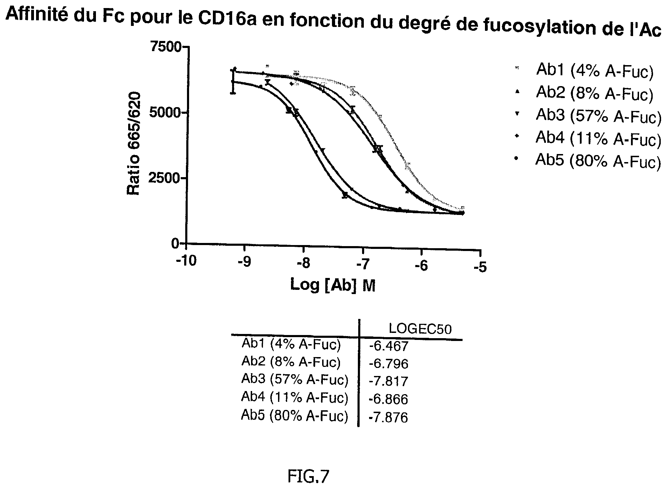

L'exemple 2 a été reproduit mais cette fois l'anticorps IgG1 non-marqué a été remplacé par différents anticorps ayant fait l'objet d'un traitement visant à éliminer les résidus fucose (Ab1 qui a subi une défucosylation de 4%, Ab2 qui a subi une défucosylation de 8%, Ab3 qui a subi une défucosylation de 57, Ab4 qui a subi une défucosylation de 11%, Ab5 qui a subi une défucosylation de 80%).
Après ajout de différentes concentrations de ces anticorps, le milieu réactionnel a été incubé pendant 3h30, et la plaque a été lue sur l'Artemis 101 (Berthold technologies) en mode HTRF®.
Il est connu que la défucosylation a pour effet d'augmenter l'affinité des anticorps pour le récepteur CD16a, et ce résultat est confirmé par la figure 7, qui montre également que l'invention permet de manière relativement simple, d'évaluer le niveau de fucosylation d'anticorps d'intérêt à partir des résultats obtenus avec des anticorps dont le niveau de fucosylation est connu. Par ailleurs, cet exemple montre également que le procédé selon l'invention est suffisamment sensible et permet de déterminer des différences d'affinité entre des anticorps ayant subi divers degrés de défucosylation.

## Revendications

1. Procédé *in vitro* de détermination de la liaison d'un anticorps compétiteur avec un récepteur Fc exprimé dans des membranes cellulaires ou des cellules intactes présentes dans un milieu de mesure, par compétition avec un anticorps de référence, comprenant les étapes suivantes :
(i) marquage direct dudit récepteur Fc par le premier membre d'un couple de partenaires de FRET, ou bien introduction dans le milieu de membranes cellulaires ou de cellules intactes dont les récepteurs Fc ont été préalablement marqués de manière directe par le premier membre d'un couple de partenaires de FRET ;
(ii) ajout dans le milieu de mesure de l'anticorps compétiteur ;
(iii)ajout dans le milieu de mesure d'un anticorps de référence, marqué de manière directe par le second membre dudit couple de partenaires de FRET ;
(iv) mesure du signal de FRET, une diminution du signal mesuré en présence de l'anticorps compétiteur par rapport à celui mesuré en son absence étant représentative de la liaison de cet anticorps au récepteur Fc ;
**caractérisé en ce que** le récepteur Fc est marqué de manière directe via une enzyme suicide, à savoir qu'il est exprimé sous forme d'une protéine de fusion comprenant le récepteur Fc et l'enzyme suicide, et que le marquage est effectué par ajout au milieu de mesure du substrat de l'enzyme, conjugué à un des partenaires de FRET.

2. Procédé selon la revendication 1, **caractérisé en ce qu'**on reproduit l'étape (ii) avec différentes quantités d'anticorps compétiteur, la quantité d'anticorps de référence ajouté à l'étape (iii) étant fixe, et **en ce qu'**il comprend une étape additionnelle (v) de détermination de l'EC50 de l'anticorps compétiteur et de comparaison avec l'EC50 de l'anticorps de référence.

3. Procédé selon les revendications 1 et 2, **caractérisé en ce que** l'anticorps de référence et l'anticorps compétiteur diffèrent notamment **en ce que** ce dernier a été fabriqué selon un procédé, ou bien a fait l'objet d'un traitement, visant à altérer le niveau de glycosylation de son fragment Fc.

4. Procédé selon les revendications 1 et 2, **caractérisé en ce que** l'anticorps de référence et l'anticorps à tester diffèrent notamment **en ce que** ce dernier a été fabriqué selon un procédé, ou bien a fait l'objet d'un traitement, visant à altérer le niveau de fucosylation de son fragment Fc.

5. Procédé de détermination du niveau de glycosylation d'un anticorps comprenant les étapes suivantes :
- mise en oeuvre du procédé selon la revendication 2 en utilisant à l'étape (ii) plusieurs anticorps compétiteurs ayant des niveaux connus de glycosylation ou de déglycosylation, puis
- mise en oeuvre du procédé selon la revendication 2 en utilisant à l'étape (ii) l'anticorps dont on veut déterminer le niveau de glycosylation, et
- détermination du niveau de glycosylation de l'anticorps étudié par comparaison de son EC50 avec celle des anticorps compétiteurs dont les niveaux de glycosylation ou de déglycosylation sont connus.

6. Procédé de détermination du niveau de fucosylation d'un anticorps comprenant les étapes suivantes :
- mise en oeuvre du procédé selon la revendication 2 en utilisant à l'étape (ii) plusieurs anticorps compétiteurs ayant des niveaux connus de fucosylation ou de défucosylation puis,
- mise en oeuvre du procédé selon la revendication 4 en utilisant à l'étape (ii) un anticorps dont on veut déterminer le niveau de fucosylation ou de défucosylation, et
- détermination du niveau de fucosylation de l'anticorps étudié par comparaison de son EC50 avec celle des anticorps compétiteurs dont les niveaux de fucosylation ou de défucosylation sont connus.

7. Procédé selon l'une quelconque des revendications précédentes, **caractérisé en ce que** le récepteur Fc est un récepteur Fc gamma.

8. Procédé selon l'une quelconque des revendications précédentes, **caractérisé en ce que** le récepteur Fc est le récepteur CD16a ou un de ses variants.

9. Procédé selon l'une des revendications 1 à 8, **caractérisé en ce que** l'enzyme suicide est choisie parmi : les mutants de l'O6-alkylguanine DNA alkyltransférase, les mutants de la déshalogénase, ou un fragment de la protéine de transport d'acyles.

10. Procédé selon l'une quelconque des revendications précédentes, **caractérisé en ce qu'**il est mis en oeuvre avec des cellules intactes.

11. Trousses de réactifs pour la mise en oeuvre du procédé selon l'une quelconque des revendications 1 à 10, **caractérisées en ce qu'**elles comprennent un vecteur d'expression qui comprend la séquence d'ADN codant pour un récepteur Fc et la séquence d'ADN codant pour une enzyme suicide ou bien des cellules qui comprennent ledit vecteur d'expression, ainsi qu'au moins un membre d'un couple de partenaires de FRET.

12. Trousses de réactifs selon la revendication 11, **caractérisées en ce que** l'enzyme suicide est choisie parmi les mutants de la déshalogénase, un fragment de la protéine de transport d'acyles ou les mutants de l'O6-alkylguanine DNA alkyltransférase.

13. Trousses de réactifs selon la revendication 11 ou 12, **caractérisées en ce que** le récepteur Fc est le récepteur CD16a.

14. Trousses de réactifs selon l'une des revendications 11 à 13, **caractérisées en ce que** les cellules sont sous forme congelée.

## Patentansprüche

1. *In-vitro-*Verfahren zur Bestimmung der Bindung eines konkurrierenden Antikörpers mit einem Fc-Rezeptor, der in Zellmembranen oder intakten Zellen exprimiert ist, die in einem Messmedium vorhanden sind, durch Konkurrenz mit einem Bezugsantikörper, das die folgenden Schritte umfasst:
(i) direktes Markieren des Fc-Rezeptors durch das erste Element eines Paares von FRET-Partnern oder Einführung von Zellmembranen oder intakten Zellen, deren Fc-Rezeptoren im Voraus direkt durch das erste Element eines Paares von FRET-Partnern markiert wurden, in das Medium,
(ii) Beimengung des konkurrierenden Antikörpers zu dem Messmedium,
(iii) Beimengung eines durch das zweite Element des Paares von FRET-Partnern direkt markierten Bezugsantikörpers zu dem Messmedium,
(iv) Messen des FRET-Signals, wobei eine Abnahme des gemessenen Signals bei Vorhandensein des konkurrierenden Antikörpers im Vergleich zu dem bei seinem Nichtvorhandensein gemessenen für die Bindung dieses Antikörpers an den Fc-Rezeptor repräsentativ ist,
**dadurch gekennzeichnet, dass** der Fc-Rezeptor direkt über ein Selbstmord-Enzym markiert wird, das heißt, dass er in der Form eines Fusionsproteins exprimiert wird, das den Fc-Rezeptor und das Selbstmord-Enzym umfasst, und dass die Markierung durch Beimengung des mit einem der FRET-Partner konjugierten Substrats des Enzyms zu dem Messmedium durchgeführt wird.

2. Verfahren nach Anspruch 1, **dadurch gekennzeichnet, dass** der Schritt (ii) mit unterschiedlichen Mengen an konkurrierendem Antikörper wiederholt wird, wobei die Menge an Bezugsantikörper, die im Schritt (iii) beigemengt wird, fest ist, und dadurch, dass es einen zusätzlichen Schritt (v) zum Bestimmen der EC50 des konkurrierenden Antikörpers und Vergleichen des Bezugsantikörpers mit der EC50 umfasst.

3. Verfahren nach den Ansprüchen 1 und 2, **dadurch gekennzeichnet, dass** der Bezugsantikörper und der konkurrierende Antikörper sich insbesondere darin unterscheiden, dass dieser letztere gemäß einem Verfahren hergestellt wurde oder einer Behandlung unterzogen wurde, das/die darauf abzielt, das Glykosylierungsniveau seines Fc-Fragments zu beeinträchtigen.

4. Verfahren nach den Ansprüchen 1 und 2, **dadurch gekennzeichnet, dass** der Bezugsantikörper und der zu testende Antikörper sich insbesondere darin unterscheiden, dass dieser letztere gemäß einem Verfahren hergestellt wurde oder einer Behandlung unterzogen wurde, das/die darauf abzielt, das Fucosylierungsniveau seines Fc-Fragments zu beinträchtigen.

5. Verfahren zur Bestimmung des Glykosylierungsniveaus eines Antikörpers, das die folgenden Schritte umfasst:
- Durchführen des Verfahrens nach Anspruch 2 unter Verwendung mehrerer konkurrierender Antikörper, die bekannte Glykosylierungs- oder Deglykosylierungsniveaus aufweisen, im Schritt (ii), dann
- Durchführen des Verfahrens nach Anspruch 2 unter Verwendung des Antikörpers, dessen Glykosylierungsniveau bestimmt werden soll, im Schritt (ii), und
- Bestimmen des Glykosylierungsniveaus des untersuchten Antikörpers durch Vergleich seiner EC50 mit derjenigen der konkurrierenden Antikörper, deren Glykosylierungs- oder Deglykosylierungsniveaus bekannt sind.

6. Verfahren zur Bestimmung des Fucosylierungsniveaus eines Antikörpers, das die folgenden Schritte umfasst:
- Durchführen des Verfahrens nach Anspruch 2 unter Verwendung mehrerer konkurrierender Antikörper, die bekannte Fucosylierungs- oder Defucosylierungsniveaus aufweisen, im Schritt (ii), dann
- Durchführen des Verfahrens nach Anspruch 4 unter Verwendung eines Antikörpers, dessen Fucosylierungs- oder Defucosylierungsniveau bestimmt werden soll, im Schritt (ii), und
- Bestimmen des Fucosylierungsniveaus des untersuchten Antikörpers durch Vergleich seiner EC50 mit derjenigen der konkurrierenden Antikörper, deren Fucosylierungs- oder Defucosylierungsniveaus bekannt sind.

7. Verfahren nach einem der vorhergehenden Ansprüche, **dadurch gekennzeichnet, dass** der Fc-Rezeptor ein Fc-gamma-Rezeptor ist.

8. Verfahren nach einem der vorhergehenden Ansprüche, **dadurch gekennzeichnet, dass** der Fc-Rezeptor der CD16a-Rezeptor oder eine seiner Varianten ist.

9. Verfahren nach einem der Ansprüche 1 bis 8, **dadurch gekennzeichnet, dass** das Selbstmord-Enzym ausgewählt wird unter: den Mutanten der O6-Alkylguanin-DNA-Alkyltransferase, den Mutanten der Dehalogenase oder einem Fragment des Acyl-Transportproteins.

10. Verfahren nach einem der vorhergehenden Ansprüche, **dadurch gekennzeichnet, dass** es mit intakten Zellen durchgeführt wird.

11. Testsätze für die Durchführung des Verfahrens nach einem der Ansprüche 1 bis 10, **dadurch gekennzeichnet, dass** sie einen Expressionsvektor umfassen, der die codierende DNA-Sequenz für einen Fc-Rezeptor und die codierende DNA-Sequenz für ein Selbstmord-Enzym oder Zellen, die den Expressionsvektor umfassen, sowie mindestens ein Element eines Paares von FRET-Partnern umfasst.

12. Testsätze nach Anspruch 11, **dadurch gekennzeichnet, dass** das Selbstmord-Enzym unter den Mutanten der Dehalogenase, einem Fragment des Acyl-Transportproteins oder den Mutanten der O6-Alkylguanin-DNA-Alkyltransferase ausgewählt ist.

13. Testsätze nach Anspruch 11 oder 12, **dadurch gekennzeichnet, dass** der Fc-Rezeptor der CD16a-Rezeptor ist.

14. Testsätze nach einem der Ansprüche 11 bis 13, **dadurch gekennzeichnet, dass** die Zellen in gefrorener Form vorliegen.

## Claims

1. An *in vitro* method for determining the binding of a competing antibody with a Fc receptor expressed in cell membranes or intact cells present in a measurement medium, by competition with a reference antibody, comprising the following steps:
(i) direct labeled of said Fc receptor with the first member of a pair of FRET partners, or else introducing, into the medium, cell membranes or intact cells whose Fc receptors were labeled beforehand directly with the first member of a pair of FRET partners;
(ii) adding the competing antibody to the measurement medium;
(iii)adding a reference antibody, labeled directly with the second member of said pair of FRET partners, to the measurement medium;
(iv)measuring the FRET signal, a decrease in the signal measured in the presence of the competing antibody relative to that measured in its absence being representative of the binding of this antibody to the Fc receptor;
**characterized in that** the Fc receptor is labeled directly via a suicide enzyme, i.e. **in that** it is expressed in the form of a fusion protein comprising the Fc receptor and the suicide enzyme, and that the labeling is carried out by adding the substrate of the enzyme, conjugated with one of the FRET partners, to the measurement medium.

2. The method according to claim 1, **characterized in that** step (ii) is reproduced with different amounts of competing antibody, the amount of reference antibody added in step (iii) being fixed, and **in that** it which comprises an additional step (v) of determining the EC50 of the competing antibody and of comparison with the EC50 of the reference antibody.

3. The method according to claims 1 and 2, **characterized in that** the reference antibody and competing antibody differ notably **in that** the latter was produced by a method, or else has undergone a treatment, aiming to alter the level of glycosylation of its Fc fragment.

4. The method according to claims 1 and 2, **characterized in that** the reference antibody and the test antibody differ notably **in that** the latter was produced by a method, or else has undergone a treatment, aiming to alter the level of fucosylation of its Fc fragment.

5. A method for determining the level of glycosylation of an antibody comprising the following steps:
- carrying out the method according to claim 2 using, in step (ii), several competing antibodies having known levels of glycosylation or of deglycosylation, then
- carrying out the method according to claim 2 using, in step (ii), the antibody whose level of glycosylation is to be determined, and
- determining the level of glycosylation of the test antibody by comparing its EC50 with that of the competing antibodies having known levels of glycosylation or of deglycosylation.

6. A method for determining the level of fucosylation of an antibody comprising the following steps:
- carrying out the method according to claim 2 using, in step (ii), several competing antibodies having known levels of fucosylation or of defucosylation then,
- carrying out the method according to claim 4 using, in step (ii), an antibody whose level of fucosylation or of defucosylation is to be determined, and
- determining the level of fucosylation of the test antibody by comparing its EC50 with that of the competing antibodies having known levels of fucosylation or of defucosylation.

7. The method according to any one of the preceding claims, **characterized in** the Fc receptor is a gamma Fc receptor.

8. The method according to any one of the preceding claims, **characterized in that** the Fc receptor is the CD16a receptor or a variant thereof.

9. The method according to claims 1 to 8, **characterized in that** the suicide enzyme is selected from: the mutants of O6-alkylguanine DNA alkyltransferase, the mutants of dehalogenase, or a fragment of the acyl carrier protein.

10. The method according to any one of the preceding claims, **characterized in that** said method is carried out with intact cells.

11. Kits of reagents for performing the method according to claims 1 to 10, **characterized in that** they comprise an expression vector which comprises the DNA sequence coding for an Fc receptor and the DNA sequence coding for a suicide enzyme or else cells which comprise said expression vector and at least one member of a pair of FRET partners.

12. Kits of reagents according to claim 11, **characterized in that** the suicide enzyme is selected from the mutants of dehalogenase, a fragment of the acyl carrier protein or the mutants of O6-alkylguanine DNA alkyltransferase.

13. Kits of reagents according to claim 11 or 12, **characterized in that** the Fc receptor is the CD16a receptor.

14. Kits of reagents according to claims 11 to 13, **characterized in that** said cells are under frozen form.


### SEQUENCE LISTING

<110> Cisbio Bioassays
<120> Procédé de détermination de la glycosylation d'un anticorps
<130> 1H305110 0004 WO
<150> FR1158245
   <151> 2011-09-16
<160> 5
<170> PatentIn version 3.5
<210> 1
   <211> 6
   <212> PRT
   <213> artificial sequence
<220>
   <223> partenaire de liaison
<220>
   <221> misc_feature
   <222> (3)..(4)
   <223> Xaa can be any naturally occurring amino acid
<400> 1
   Cys Cys Xaa Xaa Cys Cys
   1               5
<210> 2
   <211> 8
   <212> PRT
   <213> artificial sequence
<220>
   <223> couple tag/antitag
<400> 2
   Asp Tyr Lys Asp Asp Asp Asp Lys
   1               5
<210> 3
   <211> 9
   <212> PRT
   <213> artificial sequence
<220>
   <223> couple tag/antitag
<400> 3
   Tyr Pro Tyr Asp Val Pro Phe Tyr Ala
   1               5
<210> 4
   <211> 6758
   <212> DNA
   <213> artificial sequence
<220>
   <223> plasmide
<220>
   <221> promoter
   <222> (232)..(819)
   <223> promoteur CMV
<220>
   <221> sig_peptide
   <222> (923)..(986)
<220>
   <221> misc_feature
   <222> (1004)..(1027)
   <223> QC tag
<220>
   <221> misc_feature
   <222> (1046)..(1591)
   <223> Snap tag
<220>
   <221> gene
   <222> (1610)..(2321)
   <223> gene d'intérêt
<220>
   <221> polyA_site
   <222> (2358)..(2582)
   <223> polyA BGH
<220>
   <221> misc_feature
   <222> (3466)..(4260)
   <223> Neo R
<220>
   <221> misc_feature
   <222> (5762)..(6622)
   <223> Ampi R
<400> 4
   gacggatcgg gagatctccc gatcccctat ggtgcactct cagtacaatc tgctctgatg      60
   ccgcatagtt aagccagtat ctgctccctg cttgtgtgtt ggaggtcgct gagtagtgcg     120
   cgagcaaaat ttaagctaca acaaggcaag gcttgaccga caattgcatg aagaatctgc     180
   ttagggttag gcgttttgcg ctgcttcgcg atgtacgggc cagatatacg cgttgacatt     240
   gattattgac tagttattaa tagtaatcaa ttacggggtc attagttcat agcccatata     300
   tggagttccg cgttacataa cttacggtaa atggcccgcc tggctgaccg cccaacgacc     360
   cccgcccatt gacgtcaata atgacgtatg ttcccatagt aacgccaata gggactttcc     420
   attgacgtca atgggtggag tatttacggt aaactgccca cttggcagta catcaagtgt     480
   atcatatgcc aagtacgccc cctattgacg tcaatgacgg taaatggccc gcctggcatt     540
   atgcccagta catgacctta tgggactttc ctacttggca gtacatctac gtattagtca     600
   tcgctattac catggtgatg cggttttggc agtacatcaa tgggcgtgga tagcggtttg     660
   actcacgggg atttccaagt ctccacccca ttgacgtcaa tgggagtttg ttttggcacc     720
   aaaatcaacg ggactttcca aaatgtcgta acaactccgc cccattgacg caaatgggcg     780
   gtaggcgtgt acggtgggag gtctatataa gcagagctct ctggctaact agagaaccca 840
   ctgcttactg gcttatcgaa attaatacga ctcactatag ggagacccaa gctggctagc 900
   gtttaaactt aagctttgag acatggcctt accagtgacc gccttgctcc tgccgctggc 960
   cttgctgctc cacgccgcca ggccggccgc cgctagcggc atcgactaca aggacgacga 1020
   tgacaaggcc ggcatcgatg ccatcatgga caaagactgc gaaatgaagc gcaccaccct 1080
   ggatagccct ctgggcaagc tggaactgtc tgggtgcgaa cagggcctgc acgagatcaa 1140
   gctgctgggc aaaggaacat ctgccgccga cgccgtggaa gtgcctgccc cagccgccgt 1200
   gctgggcgga ccagagccac tgatgcaggc caccgcctgg ctcaacgcct actttcacca 1260
   gcctgaggcc atcgaggagt tccctgtgcc agccctgcac cacccagtgt tccagcagga 1320
   gagctttacc cgccaggtgc tgtggaaact gctgaaagtg gtgaagttcg gagaggtcat 1380
   cagctaccag cagctggccg ccctggccgg caatcccgcc gccaccgccg ccgtgaaaac 1440
   cgccctgagc ggaaatcccg tgcccattct gatcccctgc caccgggtgg tgtctagctc 1500
   tggcgccgtg gggggctacg agggcgggct cgccgtgaaa gagtggctgc tggcccacga 1560
   gggccacaga ctgggcaagc ctgggctggg tgatatccag cacagtggcg gccgcgaaga 1620
   tctcccaaag gctgtggtgt tcctggagcc tcaatggtac agggtgctcg agaaggacag 1680
   tgtgactctg aagtgccagg gagcctactc ccctgaggac aattccacac agtggtttca 1740
   caatgagagc ctcatctcaa gccaggcctc gagctacttc attgacgctg ccacagtcga 1800
   cgacagtgga gagtacaggt gccagacaaa cctctccacc ctcagtgacc cggtgcagct 1860
   agaagtccat atcggctggc tgttgctcca ggcccctcgg tgggtgttca aggaggaaga 1920
   ccctattcac ctgaggtgtc acagctggaa gaacactgct ctgcataagg tcacatattt 1980
   acagaatggc aaaggcagga agtattttca tcataattct gacttctaca ttccaaaagc 2040
   cacactcaaa gacagcggct cctacttctg cagggggctt gtcgggagta aaaatgtgtc 2100
   ttcagagact gtgaacatca ccatcactca aggtttggca gtgtcaacca tctcatcatt 2160
   ctttccacct gggtaccaag tctctttctg cttggtgatg gtactccttt ttgcagtgga 2220
   cacaggacta tatttctctg tgaagacaaa cattcgaagc tcaacaagag actggaagga 2280
   ccataaattt aaatggagaa aggaccctca agacaaatga tctagagggc ccgtttaaac 2340
   ccgctgatca gcctcgactg tgccttctag ttgccagcca tctgttgttt gcccctcccc 2400
   cgtgccttcc ttgaccctgg aaggtgccac tcccactgtc ctttcctaat aaaatgagga 2460
   aattgcatcg cattgtctga gtaggtgtca ttctattctg gggggtgggg tggggcagga 2520
   cagcaagggg gaggattggg aagacaatag caggcatgct ggggatgcgg tgggctctat 2580
   ggcttctgag gcggaaagaa ccagctgggg ctctaggggg tatccccacg cgccctgtag 2640
   cggcgcatta agcgcggcgg gtgtggtggt tacgcgcagc gtgaccgcta cacttgccag 2700
   cgccctagcg cccgctcctt tcgctttctt cccttccttt ctcgccacgt tcgccggctt 2760
   tccccgtcaa gctctaaatc gggggctccc tttagggttc cgatttagtg ctttacggca 2820
   cctcgacccc aaaaaacttg attagggtga tggttcacgt agtgggccat cgccctgata 2880
   gacggttttt cgccctttga cgttggagtc cacgttcttt aatagtggac tcttgttcca 2940
   aactggaaca acactcaacc ctatctcggt ctattctttt gatttataag ggattttgcc 3000
   gatttcggcc tattggttaa aaaatgagct gatttaacaa aaatttaacg cgaattaatt 3060
   ctgtggaatg tgtgtcagtt agggtgtgga aagtccccag gctccccagc aggcagaagt 3120
   atgcaaagca tgcatctcaa ttagtcagca accaggtgtg gaaagtcccc aggctcccca 3180
   gcaggcagaa gtatgcaaag catgcatctc aattagtcag caaccatagt cccgccccta 3240
   actccgccca tcccgcccct aactccgccc agttccgccc attctccgcc ccatggctga 3300
   ctaatttttt ttatttatgc agaggccgag gccgcctctg cctctgagct attccagaag 3360
   tagtgaggag gcttttttgg aggcctaggc ttttgcaaaa agctcccggg agcttgtata 3420
   tccattttcg gatctgatca agagacagga tgaggatcgt ttcgcatgat tgaacaagat 3480
   ggattgcacg caggttctcc ggccgcttgg gtggagaggc tattcggcta tgactgggca 3540
   caacagacaa tcggctgctc tgatgccgcc gtgttccggc tgtcagcgca ggggcgcccg 3600
   gttctttttg tcaagaccga cctgtccggt gccctgaatg aactgcagga cgaggcagcg 3660
   cggctatcgt ggctggccac gacgggcgtt ccttgcgcag ctgtgctcga cgttgtcact 3720
   gaagcgggaa gggactggct gctattgggc gaagtgccgg ggcaggatct cctgtcatct 3780
   caccttgctc ctgccgagaa agtatccatc atggctgatg caatgcggcg gctgcatacg 3840
   cttgatccgg ctacctgccc attcgaccac caagcgaaac atcgcatcga gcgagcacgt 3900
   actcggatgg aagccggtct tgtcgatcag gatgatctgg acgaagagca tcaggggctc 3960
   gcgccagccg aactgttcgc caggctcaag gcgcgcatgc ccgacggcga ggatctcgtc 4020
   gtgacccatg gcgatgcctg cttgccgaat atcatggtgg aaaatggccg cttttctgga 4080
   ttcatcgact gtggccggct gggtgtggcg gaccgctatc aggacatagc gttggctacc 4140
   cgtgatattg ctgaagagct tggcggcgaa tgggctgacc gcttcctcgt gctttacggt 4200
   atcgccgctc ccgattcgca gcgcatcgcc ttctatcgcc ttcttgacga gttcttctga 4260
   gcgggactct ggggttcgaa atgaccgacc aagcgacgcc caacctgcca tcacgagatt 4320
   tcgattccac cgccgccttc tatgaaaggt tgggcttcgg aatcgttttc cgggacgccg 4380
   gctggatgat cctccagcgc ggggatctca tgctggagtt cttcgcccac cccaacttgt 4440
   ttattgcagc ttataatggt tacaaataaa gcaatagcat cacaaatttc acaaataaag 4500
   catttttttc actgcattct agttgtggtt tgtccaaact catcaatgta tcttatcatg 4560
   tctgtatacc gtcgacctct agctagagct tggcgtaatc atggtcatag ctgtttcctg 4620
   tgtgaaattg ttatccgctc acaattccac acaacatacg agccggaagc ataaagtgta 4680
   aagcctgggg tgcctaatga gtgagctaac tcacattaat tgcgttgcgc tcactgcccg 4740
   ctttccagtc gggaaacctg tcgtgccagc tgcattaatg aatcggccaa cgcgcgggga 4800
   gaggcggttt gcgtattggg cgctcttccg cttcctcgct cactgactcg ctgcgctcgg 4860
   tcgttcggct gcggcgagcg gtatcagctc actcaaaggc ggtaatacgg ttatccacag 4920
   aatcagggga taacgcagga aagaacatgt gagcaaaagg ccagcaaaag gccaggaacc 4980
   gtaaaaaggc cgcgttgctg gcgtttttcc ataggctccg cccccctgac gagcatcaca 5040
   aaaatcgacg ctcaagtcag aggtggcgaa acccgacagg actataaaga taccaggcgt 5100
   ttccccctgg aagctccctc gtgcgctctc ctgttccgac cctgccgctt accggatacc 5160
   tgtccgcctt tctcccttcg ggaagcgtgg cgctttctca tagctcacgc tgtaggtatc 5220
   tcagttcggt gtaggtcgtt cgctccaagc tgggctgtgt gcacgaaccc cccgttcagc 5280
   ccgaccgctg cgccttatcc ggtaactatc gtcttgagtc caacccggta agacacgact 5340
   tatcgccact ggcagcagcc actggtaaca ggattagcag agcgaggtat gtaggcggtg 5400
   ctacagagtt cttgaagtgg tggcctaact acggctacac tagaagaaca gtatttggta 5460
   tctgcgctct gctgaagcca gttaccttcg gaaaaagagt tggtagctct tgatccggca 5520
   aacaaaccac cgctggtagc ggtttttttg tttgcaagca gcagattacg cgcagaaaaa 5580
   aaggatctca agaagatcct ttgatctttt ctacggggtc tgacgctcag tggaacgaaa 5640
   actcacgtta agggattttg gtcatgagat tatcaaaaag gatcttcacc tagatccttt 5700
   taaattaaaa atgaagtttt aaatcaatct aaagtatata tgagtaaact tggtctgaca 5760
   gttaccaatg cttaatcagt gaggcaccta tctcagcgat ctgtctattt cgttcatcca 5820
   tagttgcctg actccccgtc gtgtagataa ctacgatacg ggagggctta ccatctggcc 5880
   ccagtgctgc aatgataccg cgagacccac gctcaccggc tccagattta tcagcaataa 5940
   accagccagc cggaagggcc gagcgcagaa gtggtcctgc aactttatcc gcctccatcc 6000
   agtctattaa ttgttgccgg gaagctagag taagtagttc gccagttaat agtttgcgca 6060
   acgttgttgc cattgctaca ggcatcgtgg tgtcacgctc gtcgtttggt atggcttcat 6120
   tcagctccgg ttcccaacga tcaaggcgag ttacatgatc ccccatgttg tgcaaaaaag 6180
   cggttagctc cttcggtcct ccgatcgttg tcagaagtaa gttggccgca gtgttatcac 6240
   tcatggttat ggcagcactg cataattctc ttactgtcat gccatccgta agatgctttt 6300
   ctgtgactgg tgagtactca accaagtcat tctgagaata gtgtatgcgg cgaccgagtt 6360
   gctcttgccc ggcgtcaata cgggataata ccgcgccaca tagcagaact ttaaaagtgc 6420
   tcatcattgg aaaacgttct tcggggcgaa aactctcaag gatcttaccg ctgttgagat 6480
   ccagttcgat gtaacccact cgtgcaccca actgatcttc agcatctttt actttcacca 6540
   gcgtttctgg gtgagcaaaa acaggaaggc aaaatgccgc aaaaaaggga ataagggcga 6600
   cacggaaatg ttgaatactc atactcttcc tttttcaata ttattgaagc atttatcagg 6660
   gttattgtct catgagcgga tacatatttg aatgtattta gaaaaataaa caaatagggg 6720
   ttccgcgcac atttccccga aaagtgccac ctgacgtc 6758
<210> 5
   <211> 5845
   <212> DNA
   <213> artificial sequence
<220>
   <223> plasmide
<220>
   <221> promoter
   <222> (232)..(819)
   <223> promoteur CMV
<220>
   <221> gene
   <222> (965)..(1229)
<220>
   <221> polyA_site
   <222> (1272)..(1496)
   <223> polyA BGH
<220>
   <221> misc_feature
   <222> (2363)..(3388)
   <223> Hygro R
<220>
   <221> misc_feature
   <222> (4849)..(5709)
   <223> Ampi R
<400> 5
   gacggatcgg gagatctccc gatcccctat ggtcgactct cagtacaatc tgctctgatg 60
   ccgcatagtt aagccagtat ctgctccctg cttgtgtgtt ggaggtcgct gagtagtgcg 120
   cgagcaaaat ttaagctaca acaaggcaag gcttgaccga caattgcatg aagaatctgc 180
   ttagggttag gcgttttgcg ctgcttcgcg atgtacgggc cagatatacg cgttgacatt 240
   gattattgac tagttattaa tagtaatcaa ttacggggtc attagttcat agcccatata 300
   tggagttccg cgttacataa cttacggtaa atggcccgcc tggctgaccg cccaacgacc 360
   cccgcccatt gacgtcaata atgacgtatg ttcccatagt aacgccaata gggactttcc 420
   attgacgtca atgggtggac tatttacggt aaactgccca cttggcagta catcaagtgt 480
   atcatatgcc aagtacgccc cctattgacg tcaatgacgg taaatggccc gcctggcatt 540
   atgcccagta catgacctta tgggactttc ctacttggca gtacatctac gtattagtca 600
   tcgctattac catggtgatg cggttttggc agtacatcaa tgggcgtgga tagcggtttg 660
   actcacgggg atttccaagt ctccacccca ttgacgtcaa tgggagtttg ttttggcacc 720
   aaaatcaacg ggactttcca aaatgtcgta acaactccgc cccattgacg caaatgggcg 780
   gtaggcgtgt acggtgggag gtctatataa gcagagctct ctggctaact agagaaccca 840
   ctgcttactg gcttatcgaa attaatacga ctcactatag ggagacccaa gctggctagc 900
   gtttaaactt aagcttggta ccgagctcgg atccactagt ccagtgtggt ggaattctgc 960
   agatatcatg attccagcag tggtcttgct cttactcctt ttggttgaac aagcagcggc 1020
   cctgggagag cctcagctct gctatatcct ggatgccatc ctgtttctgt atggaattgt 1080
   cctcaccctc ctctactgtc gactgaagat ccaagtgcga aaggcagcta taaccagcta 1140
   tgagaaatca gatggtgttt acacgggcct gagcaccagg aaccaggaga cttacgagac 1200
   tctgaagcat gagaaaccac cacagtagct cgagtctaga gggcccgttt aaacccgctg 1260
   atcagcctcg actgtgcctt ctagttgcca gccatctgtt gtttgcccct cccccgtgcc 1320
   ttccttgacc ctggaaggtg ccactcccac tgtcctttcc taataaaatg aggaaattgc 1380
   atcgcattgt ctgagtaggt gtcattctat tctggggggt ggggtggggc aggacagcaa 1440
   gggggaggat tgggaagaca atagcaggca tgctggggat gcggtgggct ctatggcttc 1500
   tgaggcggaa agaaccagct ggggctctag ggggtatccc cacgcgccct gtagcggcgc 1560
   attaagcgcg gcgggtgtgg tggttacgcg cagcgtgacc gctacacttg ccagcgccct 1620
   agcgcccgct cctttcgctt tcttcccttc ctttctcgcc acgttcgccg gctttccccg 1680
   tcaagctcta aatcggggca tccctttagg gttccgattt agtgctttac ggcacctcga 1740
   ccccaaaaaa cttgattagg gtgatggttc acgtagtggg ccatcgccct gatagacggt 1800
   ttttcgccct ttgacgttgg agtccacgtt ctttaatagt ggactcttgt tccaaactgg 1860
   aacaacactc aaccctatct cggtctattc ttttgattta taagggattt tggggatttc 1920
   ggcctattgg ttaaaaaatg agctgattta acaaaaattt aacgcgaatt aattctgtgg 1980
   aatgtgtgtc agttagggtg tggaaagtcc ccaggctccc caggcaggca gaagtatgca 2040
   aagcatgcat ctcaattagt cagcaaccag gtgtggaaag tccccaggct ccccagcagg 2100
   cagaagtatg caaagcatgc atctcaatta gtcagcaacc atagtcccgc ccctaactcc 2160
   gcccatcccg cccctaactc cgcccagttc cgcccattct ccgccccatg gctgactaat 2220
   tttttttatt tatgcagagg ccgaggccgc ctctgcctct gagctattcc agaagtagtg 2280
   aggaggcttt tttggaggcc taggcttttg caaaaagctc ccgggagctt gtatatccat 2340
   tttcggatct gatcagcacg tgatgaaaaa gcctgaactc accgcgacgt ctgtcgagaa 2400
   gtttctgatc gaaaagttcg acagcgtctc cgacctgatg cagctctcgg agggcgaaga 2460
   atctcgtgct ttcagcttcg atgtaggagg gcgtggatat gtcctgcggg taaatagctg 2520
   cgccgatggt ttctacaaag atcgttatgt ttatcggcac tttgcatcgg ccgcgctccc 2580
   gattccggaa gtgcttgaca ttggggaatt cagcgagagc ctgacctatt gcatctcccg 2640
   ccgtgcacag ggtgtcacgt tgcaagacct gcctgaaacc gaactgcccg ctgttctgca 2700
   gccggtcgcg gaggccatgg atgcgatcgc tgcggccgat cttagccaga cgagcgggtt 2760
   cggcccattc ggaccgcaag gaatcggtca atacactaca tggcgtgatt tcatatgcgc 2820
   gattgctgat ccccatgtgt atcactggca aactgtgatg gacgacaccg tcagtgcgtc 2880
   cgtcgcgcag gctctcgatg agctgatgct ttgggccgag gactgccccg aagtccggca 2940
   cctcgtgcac gcggatttcg gctccaacaa tgtcctgacg gacaatggcc gcataacagc 3000
   ggtcattgac tggagcgagg cgatgttcgg ggattcccaa tacgaggtcg ccaacatctt 3060
   cttctggagg ccgtggttgg cttgtatgga gcagcagacg cgctacttcg agcggaggca 3120
   tccggagctt gcaggatcgc cgcggctccg ggcgtatatg ctccgcattg gtcttgacca 3180
   actctatcag agcttggttg acggcaattt cgatgatgca gcttgggcgc agggtcgatg 3240
   cgacgcaatc gtccgatccg gagccgggac tgtcgggcgt acacaaatcg cccgcagaag 3300
   cgcggccgtc tggaccgatg gctgtgtaga agtactcgcc gatagtggaa accgacgccc 3360
   cagcactcgt ccgagggcaa aggaatagca cgtgctacga gatttcgatt ccaccgccgc 3420
   cttctatgaa aggttgggct tcggaatcgt tttccgggac gccggctgga tgatcctcca 3480
   gcgcggggat ctcatgctgg agttcttcgc ccaccccaac ttgtttattg cagcttataa 3540
   tggttacaaa taaagcaata gcatcacaaa tttcacaaat aaagcatttt tttcactgca 3600
   ttctagttgt ggtttgtcca aactcatcaa tgtatcttat catgtctgta taccgtcgac 3660
   ctctagctag agcttggcgt aatcatggtc atagctgttt cctgtgtgaa attgttatcc 3720
   gctcacaatt ccacacaaca tacgagccgg aagcataaag tgtaaagcct ggggtgccta 3780
   atgagtgagc taactcacat taattgcgtt gcgctcactg cccgctttcc agtcgggaaa 3840
   cctgtcgtgc cagctgcatt aatgaatcgg ccaacgcgcg gggagaggcg gtttgcgtat 3900
   tgggcgctct tccgcttcct cgctcactga ctcgctgcgc tcggtcgttc ggctgcggcg 3960
   agcggtatca gctcactcaa aggcggtaat acggttatcc acagaatcag gggataacgc 4020
   aggaaagaac atgtgagcaa aaggccagca aaaggccagg aaccgtaaaa aggccgcgtt 4080
   gctggcgttt ttccataggc tccgcccccc tgacgagcat cacaaaaatc gacgctcaag 4140
   tcagaggtgg cgaaacccga caggactata aagataccag gcgtttcccc ctggaagctc 4200
   cctcgtgcgc tctcctgttc cgaccctgcc gcttaccgga tacctgtccg cctttctccc 4260
   ttcgggaagc gtggcgcttt ctcaatgctc acgctgtagg tatctcagtt cggtgtaggt 4320
   cgttcgctcc aagctgggct gtgtgcacga accccccgtt cagcccgacc gctgcgcctt 4380
   atccggtaac tatcgtcttg agtccaaccc ggtaagacac gacttatcgc cactggcagc 4440
   agccactggt aacaggatta gcagagcgag gtatgtaggc ggtgctacag agttcttgaa 4500
   gtggtggcct aactacggct acactagaag gacagtattt ggtatctgcg ctctgctgaa 4560
   gccagttacc ttcggaaaaa gagttggtag ctcttgatcc ggcaaacaaa ccaccgctgg 4620
   tagcggtggt ttttttgttt gcaagcagca gattacgcgc agaaaaaaag gatctcaaga 4680
   agatcctttg atcttttcta cggggtctga cgctcagtgg aacgaaaact cacgttaagg 4740
   gattttggtc atgagattat caaaaaggat cttcacctag atccttttaa attaaaaatg 4800
   aagttttaaa tcaatctaaa gtatatatga gtaaacttgg tctgacagtt accaatgctt 4860
   aatcagtgag gcacctatct cagcgatctg tctatttcgt tcatccatag ttgcctgact 4920
   ccccgtcgtg tagataacta cgatacggga gggcttacca tctggcccca gtgctgcaat 4980
   gataccgcga gacccacgct caccggctcc agatttatca gcaataaacc agccagccgg 5040
   aagggccgag cgcagaagtg gtcctgcaac tttatccgcc tccatccagt ctattaattg 5100
   ttgccgggaa gctagagtaa gtagttcgcc agttaatagt ttgcgcaacg ttgttgccat 5160
   tgctacaggc atcgtggtgt cacgctcgtc gtttggtatg gcttcattca gctccggttc 5220
   ccaacgatca aggcgagtta catgatcccc catgttgtgc aaaaaagcgg ttagctcctt 5280
   cggtcctccg atcgttgtca gaagtaagtt ggccgcagtg ttatcactca tggttatggc 5340
   agcactgcat aattctctta ctgtcatgcc atccgtaaga tgcttttctg tgactggtga 5400
   gtactcaacc aagtcattct gagaatagtg tatgcggcga ccgagttgct cttgcccggc     5460
   gtcaatacgg gataataccg cgccacatag cagaacttta aaagtgctca tcattggaaa     5520
   acgttcttcg gggcgaaaac tctcaaggat cttaccgctg ttgagatcca gttcgatgta     5580
   acccactcgt gcacccaact gatcttcagc atcttttact ttcaccagcg tttctgggtg     5640
   agcaaaaaca ggaaggcaaa atgccgcaaa aaagggaata agggcgacac ggaaatgttg     5700
   aatactcata ctcttccttt ttcaatatta ttgaagcatt tatcagggtt attgtctcat     5760
   gagcggatac atatttgaat gtatttagaa aaataaacaa ataggggttc cgcgcacatt     5820
   tccccgaaaa gtgccacctg acgtc                                           5845